# EUROPEAN PATENT SPECIFICATION

(11) **EP 1 028 959 B1**
(45) Date of publication and mention of the grant of the patent: **27.06.2007**
(21) Application number: 98956524.7
(22) Date of filing: 04.11.1998
(51) Int. Cl.: C07D 409/12, A01N 43/40, C07D 409/14

(54) **HERBICIDAL FURANYL- AND THIENYLOXYAZINES**
HERBIZIDE FURANYL- UND THIENYLOXYAZINE
FURANYL- ET THIENYLOXYAZINES UTILISEES COMME HERBICIDES

(30) Priority: 07.11.1997 US 966486; 07.11.1997 US 966339; 07.11.1997 US 967353
(43) Date of publication of application: 23.08.2000
(73) Proprietor: BASF Aktiengesellschaft, 67056 Ludwigshafen (DE)
(72) Inventor: KARP, Gary, Mitchell, Princeton Junction, NJ 08550 (US); CONDON, Michael, Edward, Lawrenceville, NJ 08648 (US); KLEEMANN, Axel, D-63454 Hanau (DE); SCHEIBLICH, Stephan, D-55128 Mainz (DE); MAIER, Thomas, D-78333 Stockach (DE); BALTRUSCHAT, Helmut, Siegfried, D-55444 Schweppenhausen (DE)
(74) Representative: Langfinger, Klaus Dieter
(86) International application number: PCT/US1998/023426
(87) International publication number: WO 1999/024427

(56) References cited:
- EP-A- 0 572 093
- EP-A- 0 693 490
- EP-A- 0 723 960
- WO-A-94/22833
- WO-A-98/04550
- PATENT ABSTRACTS OF JAPAN vol. 016, no. 013 (C-0901), 14 January 1992 & JP 03 232884 A (KUMIAI CHEM IND CO LTD;OTHERS: 01), 16 October 1991
- PATENT ABSTRACTS OF JAPAN vol. 016, no. 098 (C-0918), 11 March 1992 & JP 03 279382 A (TOKUYAMA SODA CO LTD), 10 December 1991

## Description

### BACKGROUND OF THE INVENTION

Mankind is dependent upon field crops for food, fiber, animal feed and the like. The ever-increasing world population dictates the necessity to produce more from each hectare and, at the same time, to preserve and protect the environment and natural resources that make such production possible. Successful crop production and environmental concerns lead to an ongoing need for new and effective methods to control pestiferous plants which provide early season weed competition and late season weed flushes causing significant loss in crop quality and yield.

This invention relates to certain novel substituted thienyloxyazines, to the preparation of such compounds, to herbicidal compositions containing such compounds, and to a method of combating undesired plant growth using such compounds.

Pyridines, pyrimidines and their derivatives have many uses in the pharmaceutical area as well as in agriculture (herbicides, fungicides, acaricides, anthelmintics, bird repellents), as reagents, intermediates and chemicals for the polymer and textile industry.

Selective herbicides the active ingredients of which are pyridine derivatives, and particularly 2,6-substituted pyridines, are known from EP0572093, EP0692474 and WO 94/22833.

The European patent application EP0693490 discloses 2-phenyloxy-6-thienylmethyloxypyridines.

The European patent application EP 0694538 discloses herbicidal 2-benzyloxy-4-phenoxypyrimidines.

Herbicidal 2,6-disubstituted pyridines and 2,4-disubstituted pyrimidines are described in EP 0 723 960. Said compounds contain a heteroaryloxy group in the 2 or 6 position wherein the heteroaryl moiety contains at least one nitrogen atom.

The European patent application EP 0 447 004 discloses similar 6-phenyloxypyrid-2-carboxamides.

Although many of these disclosed pyridine and pyrimidine compounds demonstrate efficacious control of a variety of weeds, said compounds are not completely satisfactory with regard to crop selectivity and consequently may have limited application in agricultural practice.

However, disubstituted pyridine and pyrimidine derivatives containing a thienyloxy group have not yet been described.

It has now been found that the thienyloxyazine compounds of the present invention are particularly useful as crop-selective herbicidal agents.

The compounds according to the present invention combine high herbicidal activity with the necessary selectivity and enhanced soil degradation.

### SUMMARY OF THE INVENTION

We have now found that, surprisingly, thienyloxyazines show excellent herbicidal activity at low dosages combined with higher selectivity in crops than those disclosed in the aforementioned patent applications.

Accordingly, the present invention provides novel compounds of the general formula I 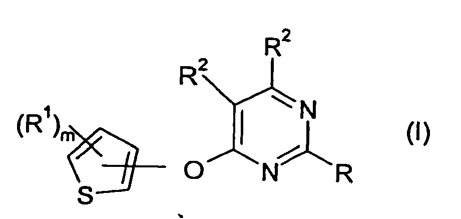 wherein
R¹ each independently represent a halogen atom; an alkyl, alkoxy, alkoxyalkyl, alkoxyalkoxy, alkoxycarbonyl or alkanoyloxy group, which may be substituted by phenyl, halogen, nitro, cyano, hydroxyl, C₁-C₄-alkoxy, C₁-C₄-haloalkoxy or C₁-C₄-alkoxycarbonyl;
an alkenyl, alkynyl, haloalkyl, haloalkoxy, formyl, cyano or SF₅-group;
a phenyl or naphthyl group, which may be substituted by halogen atoms, nitro, cyano, amino, hydroxy, phenoxy, C₁-C₄-alkyl, C₁-C₄-alkoxy, C₁-C₄-haloalkyl, C₁-C₄-haloalkenyl, C₁-C₄-haloalkoxy, C₁-C₄-haloalkylthio, C₁-C₄-alkylsulfonyl or halosulfonyl; a -S(O)ₚ-R³ group, in which p is 0, 1 or 2, and R³ represents an alkyl or haloalkyl group; -NR⁴R⁵, in which R⁴ and R⁵ each independently represent a hydrogen atom, an alkyl, alkenyl, aralkyl or aryl group;
R² each independently represent a hydrogen atom, a nitro group or has the meaning given for R¹;
R represents a group of formula Z-B, wherein
Z represents a single bond, an oxygen or sulfur atom, or a -OCH₂- or -SCH₂- group;
B represents a phenyl, naphthyl or a 5- or 6-membered heteroaryl group or a thienyl group
which may be substituted by halogen atoms, nitro, cyano, amino, hydroxy, phenoxy, C₁-C₄-alkyl, C₁-C₄-alkoxy, C₁-C₄-haloalkyl, C₁-C₄-haloalkenyl, C₁-C₄-haloalkoxy, C₁-C₄-haloalkylthio, C₁-C₄-alkylsulfonyl or halosulfanyl; and
m is 0 or an integer from 1 to 3;
wherein any of the above mentioned moietys comprises an alkyl, alkenyl or alkynyl group, such groups may be linear or branched and contain 1 to 6 carbon atoms;
wherein the alkyl portion of a haloalkyl, haloalkoxy, or alkoxy group has from 1 to 4 carbon atoms; and
wherein the number of carbon atoms in the alkoxyalkyl, alkoxyalkoxy or dialkoxyalkyl group is up to 6.

The compounds show excellent selective herbicidal activity in certain crops, such as maize and rice, and enhanced soil degradation.

The present invention also provides a compound of formula IIIA 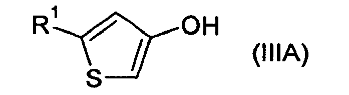 wherein R¹ is as defined hereinabove for formula I.

It is another object of the invention to provide methods for controlling undesired plant growth by contacting said plants with a herbicidally effective amount of the new compounds.

Further provided are selective herbicidal compositions and methods for the control of monocotyledenous and dicotyledenous plant species and processes to prepare herbicidal compounds of formula I employing the intermediate of formula IIIA.

Those and other objects and features of the invention will become more apparent from the detailed description set forth hereinbelow.

### DETAILED DESCRIPTION OF THE PREFERRED EMBODIMENTS

Improved crop production methods and new, effective herbicidal agents are continually being sought by agriculturalists around the world. Surprisingly, the formula I thienyloxy compounds of the invention demonstrate excellent control of a wide variety of monocotyledenous and dicotyledenous weeds. Advantageously, the formula I compounds of the invention also demonstrate crop selectivity, particularly cereal crop selectivity, and especially com selectivity.

Generally, if any of the above mentioned moieties comprises an alkyl, alkenyl or alkynyl group, such groups, unless otherwise specified, may be linear or branched and may contain 1 to 6, preferably 1 to 4, carbon atoms. Examples of such groups are methyl, ethyl, propyl, vinyl, allyl, propargyl, isopropyl, butyl, isobutyl and tertiary-butyl groups. The alkyl portion of a haloalkyl, haloalkoxy, haloalkylthio, alkylthio or alkoxy group suitably has from 1 to 4 carbon atoms, preferably 1 or 2 carbon atoms. The number of carbon atoms in the alkoxyalkyl, alkoxyalkoxy or dialkoxyalkyl groups is up to 6, preferably up to 4, e.g. methoxymethyl, methoxymethoxy, methoxyethyl, ethoxymethyl, ethoxyethoxy, dimethoxymethyl.

"Halogen" means a fluorine, chlorine, bromine or iodine atom, preferably fluorine, chlorine or bromine. Haloalkyl moieties of any groups within the definitions used herein and as such can contain one or more halogen atoms, preferably an alkyl group of formula CₙH₂ₙ₊₁, wherein from 1 to 2n +1 hydrogen atoms are replaced by a halogen atom, preferably a fluorine atom. Haloalkyl, haloalkoxy and haloalkylthio are preferably mono-, di-, tri- or perfluoroalkyl, -alkoxy and -alkylthio, especially trifluoromethyl, chlorodifluoromethyl, pentafluoroethyl, trifluoromethoxy, difluoromethoxy, difluoromethylthio, trifluoromethylthio, 2,2,2-trifluoroethyl, 1,1,1-trifluoroprop-2-yl or 2,2,2-trifluoroethoxy groups.

R represents a group of formula -Z-B, wherein Z represents -O-, -S-, -OCH₂-, -SCH₂-, or a single bond, and B represents a phenyl, naphthyl or a 5- or 6-membered heteroaryl group or a thienyl group
which may be substituted by halogen atoms, nitro, cyano, amino, hydroxy, phenoxy, C₁-C₄-alkyl, C₁-C₄-alkoxy, C₁-C₄-haloalkyl, C₁-C₄-haloalkenyl, C₁-C₄-haloalkoxy, C₁-C₄-haloalkylthio, C₁-C₄-alkylsulfonyl or halosulfanyl.

In the event that Z represents a simple bond B is preferably a phenyl group,
which may be substituted by halogen atoms, nitro, cyano, amino, hydroxy, phenoxy, C₁-C₄-alkyl, C₁-C₄-alkoxy, C₁-C₄-haloalkyl, C₁-C₄-haloalkenyl, C₁-C₄-haloalkoxy, C₁-C₄-haloalkylthio, C₁-C₄-alkylsulfonyl or halosulfanyl.

An aryl group as substituent or part of other substituents is suitably an optionally substituted phenyl or naphthyl group. An heteroaryl group as substituent or part of other substituents is suitably an optionally substituted 5- or 6-membered heterocyclus containing one or more heteroatoms selected from nitrogen, oxygen and sulfur, at least 1 oxygen, 1 sulfur atom or 1 to 3 nitrogen atoms being preferred. Examples of such groups are thienyl, furanyl, pyrazolyl, imidazolyl, triazolyl, tetrazolyl, thiazolyl, pyridyl, pyrazinyl, pyrimidyl, pyridazinyl, and triazinyl groups. 5- or 6-membered heterocycles may be fused with a benzene ring.

"Aryl" and "heteroaryl" preferably represent a phenyl, pyridyl, thienyl or pyrazolyl group being substituted by one or more of the same or different substituents selected from halogen atoms, alkyl groups, alkoxy groups, cyano groups, haloalkyl groups, haloalkoxy groups, alkylthio groups, haloalkylthio groups and SF₅ groups.

When any groups are designated as being optionally substituted, the substituent groups which are optionally present may be any of those customarily employed in the modification and/or development of pesticidal compounds and are especially substituents that maintain or enhance the herbicidal activity associated with the compounds of the present invention, or influence persistence of action, soil or plant penetration, or any other desirable property of such herbicidal compounds.

There may be one or more of the same or different substituents present in each part of the molecules. In relation to moieties defined above as comprising an optionally substituted alkyl group, including alkyl parts of haloalkyl, alkoxy, alkylthio, haloalkoxy, alkylamino and dialkylamino groups, specific examples of such substituents include phenyl, halogen atoms, nitro, cyano, hydroxyl, C₁₋₄-alkoxy, C₁₋₄-haloalkoxy and C₁₋₄-alkoxycarbonyl groups.

In relation to moieties defined above as comprising an optionally substituted aryl or heteroaryl group, optional substituents include halogen, especially fluorine, chlorine and bromine atoms, and nitro, cyano, amino, hydroxy, phenoxy, C₁₋₄-alkyl, C₁₋₄-alkoxy, C₁₋₄-haloalkyl, C₁₋₄-haloalkenyl, C₁₋₄-haloalkoxy, C₁₋₄-haloalkylthio, C₁₋₄-alkylsulfonyl and halosulfanyl groups such as SF₅. In the case of phenyl-groups 1 to 5 substituents may suitably be employed, in the case of thienyl-groups 1 to 3 substituents may suitably be employed, 1 or 2 substituents being preferred.

Typically haloalkyl, haloalkoxy and haloalkylthio groups are trifluoromethyl, pentafluoroethyl, 2,2,2-trifluoroethyl, 1,1,1-trifluoroprop-2-yl, trifluoromethoxy, difluoromethoxy, 2,2,2-trifluoroethoxy and trifluoromethylthio groups.
R¹ is preferably a halogen atom,
a C₁-C₆alkyl group optionally substituted with one or more halogen atoms, C₁-C₄alkoxy, C₁-C₄haloalkoxy. C₁-C₄alkoxycarbonyl. OH, CN, NO₂, or phenyl groups,
a C₂-C₆alkenyl group
a C₃-C₆alkynyl group
a C₁-C₆alkoxy group optionally substituted with one or more halogen atoms, C₁-C₄alkoxy, C₁-C₄haloalkoxy, C₁-C₄alkoxycarbonyl, OH, CN, NO₂, or phenyl groups,
a phenyl group optionally substituted with one or more halogen atoms, C₁-C₄alkoxy, C₁-C₄haloalkoxy, - OH, CN, NO₂, C₁-C₄-alkylsulfonyl, or phenyl groups, or
a R¹⁰S(O)_{y}, NR¹¹R¹², CN, CX'R⁹ group, in which X' is O;
R⁹ is H;
R¹⁰ is C₁-C₄-alkyl;
R¹¹ and R¹² are each independently H or C₁-C₄-alkyl;
and
y is each independently 0 or an integer of 1 or 2.
R² is preferably a hydrogen or halogen atom,
a C₁-C₆alkyl group optionally substituted with one or more halogen atoms, C₁-C₄alkoxy, C₁-C₄haloalkoxy, C₁-C₄alkoxycarbonyl, OH, CN, NO₂, or phenyl groups,
a C₂-C₆alkenyl group
a C₃-C₆alkynyl group
a C₁-C₆alkoxy group optionally substituted with one or more halogen atoms, C₁-C₄alkoxy, C₁-C₄haloalkoxy, C₁-C₄alkoxycarbonyl, OH, CN, NO₂, or phenyl groups, or
a R¹⁰S(O)_{y}, NR¹¹R¹², CN, CX'R⁹ group, in which X' is O ;
R⁹ is H;
R¹⁰ is C₁-C₄-alkyl;
R¹¹ and R¹² are each independently H or C₁-C₄-alkyl; and
y is each independently 0 or an integer of 1 or 2.
R³ is preferably a C₁-C₆alkyl group optionally substituted with one or more halogen atoms, preferably fluorine atoms.

R⁴ and R⁵ are each independently preferably a hydrogen atom, a C₁-C₆alkyl group, a C₂-C₆alkenyl group or a phenyl group.

Preferably m is 0, 1 or 2, in particular 1. When m is at least 1, one substituent R¹ is most preferably located at the 5-position.

The thienyl group may be attached in the 2- or 3-position with respect to the oxygen or sulfur atom. 3-thienyl groups are preferred.

Particularly preferred are the compounds of formula IA. 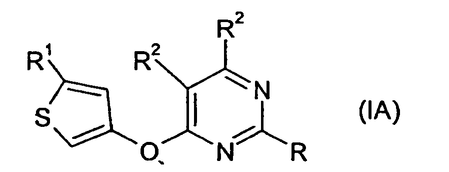 wherein R¹, R² and R have the meaning given hereinabove.

In formula I R preferably represents a group of formula -Z-B, in which Z represents -O- or -OCH₂- and B represents a group selected from the formulae a, b, c or d: 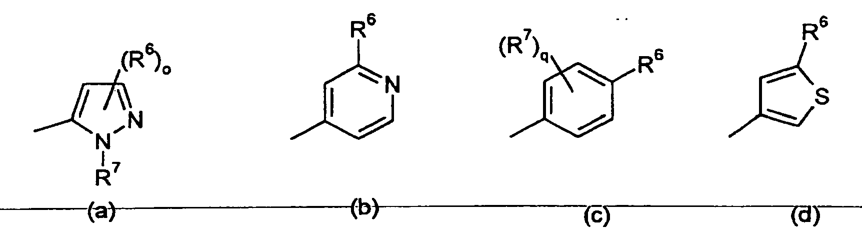 wherein
R⁶ and R⁷ each independently represent a hydrogen atom or halogen atom or an optionally substituted alkyl or alkoxy group, o is 1 or 2 and q is 1, 2, 3 or 4.
R⁶ and R⁷ are each independently preferably a hydrogen atom or halogen atom,
a C₁-C₆alkyl group optionally substituted with one or more halogen atoms,
or
a C₁-C₆alkoxy group optionally substituted with one or more halogen atoms,

Particularly preferred are the compounds of formula IA1 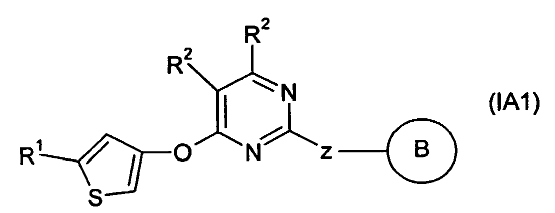 wherein B has the meaning given and Z is -O- or -OCH₂.

Suitably, R¹ represents a halogen atom or an optionally substituted alkyl, alkoxy, alkyl thio or cyano group.

Preferably, R₁ represents an optionally substituted C₁₋₆ alkyl, C₁₋₆ alkoxy or C₁₋₆ alkylthio group which is unsubstituted, or substituted by one or more moieties independently selected from halogen atoms. In particular R¹ denotes a fluorine or chlorine atom or a methyl, ethyl, iso-propyl or *tert-*butyl, methoxy, trifluoromethyl, perfluoroethyl, trifluormethoxy, difluoromethoxy, trifluormethylthio or difluoromethylthio group.

Preferably, R² represents a cyano group or a C₁₋₆ alkyl, C₁₋₆ alkoxy or C₁₋₆ alkylthio group which is unsubstituted, or substituted by one or more moieties independently selected from halogen atoms. In particular R² denotes a fluorine or chlorine atom or a methyl, ethyl, methoxy, methylthio or a cyano group.

The invention is exemplified by the following specific compounds:
2,4-bis(5-trifluoromethylthienyl-3-oxy)-6-methoxypyrimidine, 2-benzyloxy-6-methoxy-4-(5-trifluoromethylthienyl-3-oxy)-pyrimidine,

In formula I R preferably represents a group of formula -Z-B, in which Z represents -single bond and B represents a group selected from the formulae c, d or e: 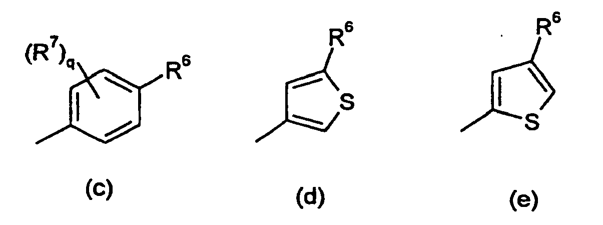 wherein
R⁶ and R⁷ each independently represent a hydrogen atom or halogen atom or an optionally substituted alkyl or alkoxy group, o is 1 or 2 and q is 1, 2, 3 or 4.

Particularly preferred are the compounds of formula 12, 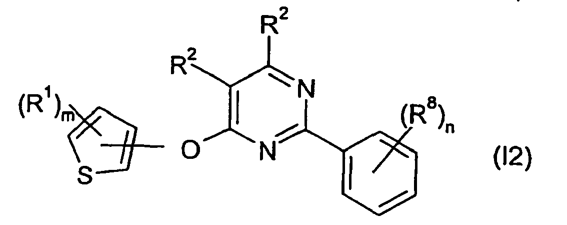 R¹, R² and m are as defined in any of claims 1 to 3;
n is 0 or an integer from 1 to 5;
R⁸ each independently represent a halogen atom,
a C₁-C₆alkyl group optionally substituted with one or more halogen atom, C₁-C₄alkoxy, C₁-C₄haloalkoxy. C₁-C₄alkoxycarbonyl, OH, CX'R⁹, CN, NO₂, R¹⁰S(O)_{y} or phenyl groups,
a C₂-C₆alkenyl group optionally substituted with one or more halogen atom, C₁-C₄alkoxy, C₁-C₄haloalkoxy, C₁-C₄alkoxycarbonyl, OH, CX'R⁹, CN, NO₂, R¹⁰S(O)_{y} or phenyl groups,
a C₃-C₆alkynyl group optionally substituted with one or more halogen atom, C₁-C₄alkoxy, C₁-C₄haloalkoxy, C₁-C₄alkoxycarbonyl, OH, CX'R⁹, CN, NO₂, R¹⁰S(O)_{y} or phenyl groups,
a C₁-C₆alkoxy group optionally substituted with one or more halogen atom, C₁-C₄alkoxy, C₁-C₄haloalkoxy, C₁-C₄alkoxycarbonyl, OH, CX'R⁹, CN, NO₂, R¹⁰S(O)_{y} or phenyl groups,
R¹⁰S(O)_{y}, NR¹¹R¹², NO₂, CN, CX'R⁹, CO₂R¹³ or OCOR¹⁴;
X' is each independently O, NOR¹⁵ or NNR¹⁶R¹⁷;
R⁹, R¹³, R¹⁴ and R¹⁵ are each independently H or C₁-C₄alkyl optionally substituted with one or more halogen, phenyl or furyl groups;
R¹⁰, R¹¹, R¹² , R¹⁶ and R¹⁷ are each independently H, C₁-C₄alkyl or C,-C₄alkoxy;
y is each independently 0 or an integer of 1 or 2.

In particular preferred are the compounds of formula IA2 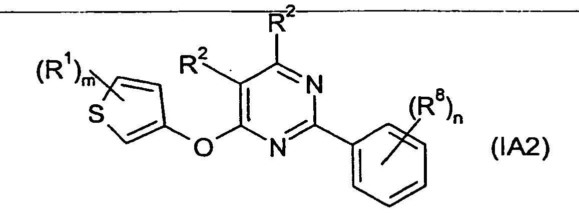 wherein R¹, R², R⁸, m and n are as defined hereinabove, in particular
wherein m is 1, and R¹ is attached to the 5-position of the thienyloxy ring; n is 1; and R⁸ is attached to the 4-position of the phenyl group.

Another group of preferred compounds having the structure of formula 12 are those compounds wherein R¹ is C₁-C₄haloalkyl,
C₁-C₄alkylsulfonyl or C₁-C₄haloalkylsulfonyl, preferably CF₃, COCF₃, CF₃SO₂, CH₃SO₂, CF₂Cl or CHF₂; R² are each independently H, halogen, C₁-C₄alkoxy or C₁-C₄alkyl optionally substituted with one or more C₁-C₄alkoxy groups; and R⁸ is CF₃.

Examples of specific compounds of formula I2 include, but are not limited to:
4-methyl-2-(α,α,α-trifluoro-p-tolyl)-6-{[5-(trifiuoromethyl)-3-thienyl]oxy}-pyrimidine; 4-methoxy-2-(α,α,α-trifluoro-p-tolyl)-6-{[5-(trifluoromethyl-3-thienyl]oxy}pyrimidine; 5-ethyl-2-(α,α,α-trifluoro-p-tolyl)-6-{[5-(trifluoromethyl-3-thienyl]oxy}pyrimidine; 4-methyl-6-(α,α,α-trifluoro-p-tolyl)-2-{[5-(trifluoromethyl)-3-thienyl]oxy}pyrimidine; 5-methyl-2-(α,α,α-trifluoro-p-tolyl)-6-{[5-(trifluoromethyl-3-thienyl]oxy}pyrimidine; 5-ethyl-2-(α,α,α-trifluoro-p-tolyl)-6-{[5-(trifluoromethyl-sulfonyl)3-thienyl]oxy}pyrimidine; 4-ethyl-2-(α,α,α-trifluoro-p-totyl)-6-{[5-(trifluoromethyl-3-thienyl]oxy}pyrimidine; 2-(α,α,α-trifluoro-p-tolyl)-6-{[5-(trifluoromethyl-3-thienyl]oxy}pydmidine; 4-n-propyl-2-(α,α,α-trifluoro-p-tolyl)-6-{[5-(trifluoromethyl-3-thienyl]oxy}pyrimidine; 4-chtoro-5-ethyl-2-(α,α,α-trifluoro-p-tolyl)-6-{[5-(trifluoromethyl-3-thienyl]oxy}pyrimidine; 4-chloro-2-(α,α,α-trifluoro-p-tolyl)-6-{[5-(trifluoromethyl-3-thienyl]oxy}pyrimidine; 4-(methoxymethy))-2-(α,α,α-trifluoro-p-totyl)-6-{[5-(trifluoromethyl-3-thienyl]oxy}pyrimidine; 4-methyl-2-(α,α,α-trifluoro-p-tolyl)-6-{[5-(hydroxymethyl)-3-thienyl]oxy}pyrimidine; 4-(difluoromethyl)-2-(α,α,α-trifluoro-p-tolyl)-6-{[5-(trifluoromethyl-3-thienyl]oxy}pyrimidine;
5-ethyl-2-(α,α,α-trifluoro-p-tolyl)-6-{[5-(trifluoromethylsulfonyl)-3-thienyl]oxy}pyrimidine; 5-methyl-2-(α,α,α-trifluoro-p-tolyl)-6-{[5-(methylsulfonyl)-3-thienyl]oxy}pyrimidine; 4-methoxy-2-(α,α,α-trifluoro-p-tofyl)-6-[(5-formyl-3-thienyl)oxy]pyrimidine; 4-ethyl-2-(α,α,α-trifluoro-p-tolyl)-6-{[5-(methylsulfonyl)-3-thienyl]oxy}pyrimidine; 4-methyl-2-(α,α,α-trifluoro-p-tolyl)-6-{[5-(trifluoromethylsulfonyl)-3-thienyl]oxy}pyrimidine; 4-cyano-2-(α,α,α-trifluoro-p-tolyl)-6-{[5-(methylsulfonyl)-3-thienyl]oxy}pyrimidine; 5-methoxymethyl-2-(α,α,α-trifluoro-*p*-tolyl)-6-{[5-(trifluoromethylsulfonyl)-3-thienyl]oxy}pyrimidine, 4-fluoro-2-(α,α,α-trifluoro-p-tolyl)-6-{[5-(trifluoromethyl)-3-thienyl]oxy}pyrimidine, 4-methytamino-2-(α,α,α-trifluoro-p-tolyl)-6-{[5-(trifluoromethyl)-3-thienyl]oxy}pyrimidine, 4-methyl-2-(4-chlorophenyl)-6-{[5-(trifluoromethyl)-3-thienyl]oxy}pyrimidine, 5-methyl-4-fluoro-2-(α,α,α-trifluoro-p-tolyl)-6-{[5-(trifluoromethyl)-3-thienyl]oxy}pyrimidine, 5-methyl-2-(4-chlorophenyl)-6-{[5-(trifluoromethyl)-3-thienyl]oxy}pyrimidine, 2-(4-chlorophenyl)-6-{[5-(trifluoromethyl)-3-thienyl]oxy}pyrimidine, 4-methyl-2-(4-tert-butylphenyl)-6-{[5-(trifluoromethyl)-3-thienyl]oxy}pyrimidine,
4-methyl-2-(3-thienyl)-6-{[5-(trifluoromethyl)-3-thienyl]oxy}pyrimidine, 5-methyl-2-(2-thienyl)-6-([5-(trifluoromethyl)-3-thienyl]oxy}pyrimidine, 2-(5-chloro-2-thienyl)-6-{[5-(trifluoromethyl)-3-thienyl]oxy}pyrimidine, 4-methoxy-2-(5-chloro-3-thienyl)-6-{[5-(trifluoromethyl)-3-thienyl]oxylpyrimidine, 4-methyl-2-(5-trifluoromethyl-2-thienyl)-6-{[5-(triftuoromethyl)-3-thienyl]oxy}pynmidine. 2-(5-trifluoromethyl-3-thienyl)-6-{[5-(trifluoromethyl)-3-thienyl]oxy}pyrimidine.

The compounds are oils, gums, or crystalline solid materials. They are superior through their valuable herbicidal properties. For example, they can be used in agriculture or related fields for the control of undesired plants. The compounds of general formula I according to the invention possess a high herbicidal activity within a wide concentration range and at low dosages, and may be used in agriculture without any difficulties, in particular for the selective control of undesired plants such as *Alopecurus myosuroides, Echinochloa crus-galli, Setaria viridis, Galium aparine, Stellaria media, Veronica persica, Lamium purpureum, Viola arvensis, Abutilon theophrasti, Ipomoea purpurea* and *Amaranthus retroflexus Panicum dichotomiflorum* by pre- and post-emergence application, particularly in certain crops, such as maize, cotton, wheat, and rice.

The compounds of formula according to the present invention can be prepared by conventional methods, particularly as follows:
reacting a compound of formula II 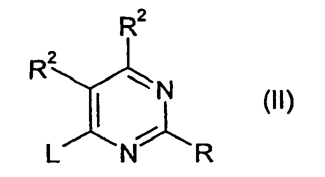
in which R is as defined hereinabove for formula I or I2, and L represents a leaving group, with a compound of formula III or a metal salt thereof 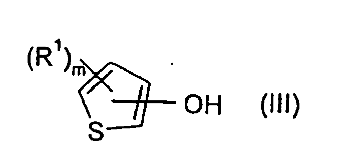 in which R¹ and m are as defined hereinabove for formula I.
(A) A suitable process for the preparation of the compounds of general formula I comprises the reaction of a compound of formula IV: 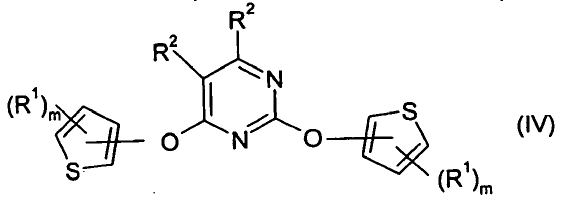 in which R¹, R² and m have the meaning given, with a compound of general formula V, 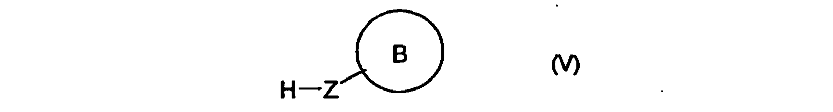 in which B and Z have the meaning given, or a metal salt thereof.
(B) The compounds of formula IV may be prepared from the compounds of the general formula VI. 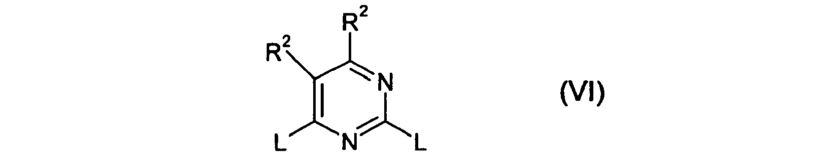 in which L is a leaving group with a compound of formula III,
   or a metal salt thereof.
(C) A suitable process for the preparation of the compounds of general formula I comprises the reaction of a compound of formula VII: 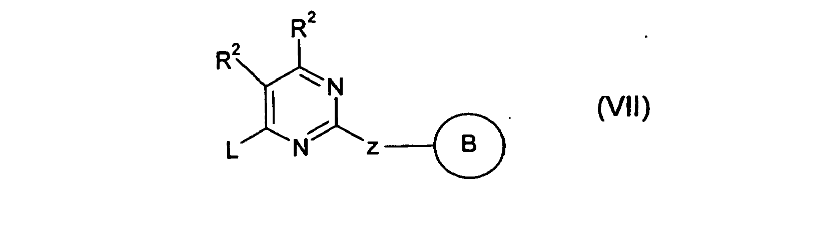 in which B and Z have the meaning given and L is a leaving group, with a compound of general formula III or a metal salt thereof.
(D) The compounds of formula I2 may be prepared by reacting a compound of formula II2 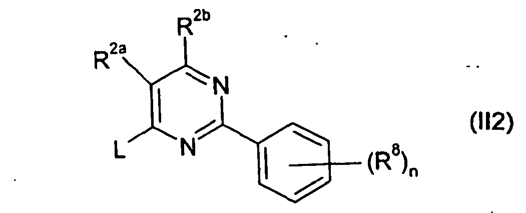 wherein L is F, Cl or Br, and R⁸ and n are as described for formula I2 and R^{2a} and R^{2b} have the meaning given for R², with at least one molar equivalent of a compound of formula III, in the presence of a base and optionally a solvent.
(E) Alternatively, compounds of formula 12 may be prepared by reacting a compound of formula II2 wherein R^{2b} is F, Cl or Br with at least 2 molar equivalents of a compound of formula III in the presence of a base and optionally a solvent to form a dithienyl(or furyl)oxy or -thioxy intermediate compound of formula VIII 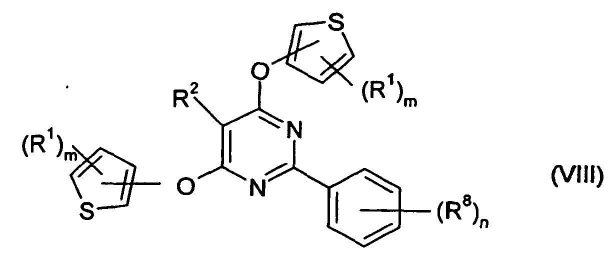
wherein R¹, R², R⁸, m and n are as described for formula 12. Using conventional methods, the 4-thienyloxy group of the intermediate formula VIII compound may be displaced by an alkanol to give compounds of formula I wherein R² is alkoxy, thio, or hydrolytically cleaved to give compounds of formula I wherein R² is hydrogen. The reaction scheme is shown in flow diagram I, wherein the alkanol or alkylthiol is an alkanol and the alkanol is methanol.

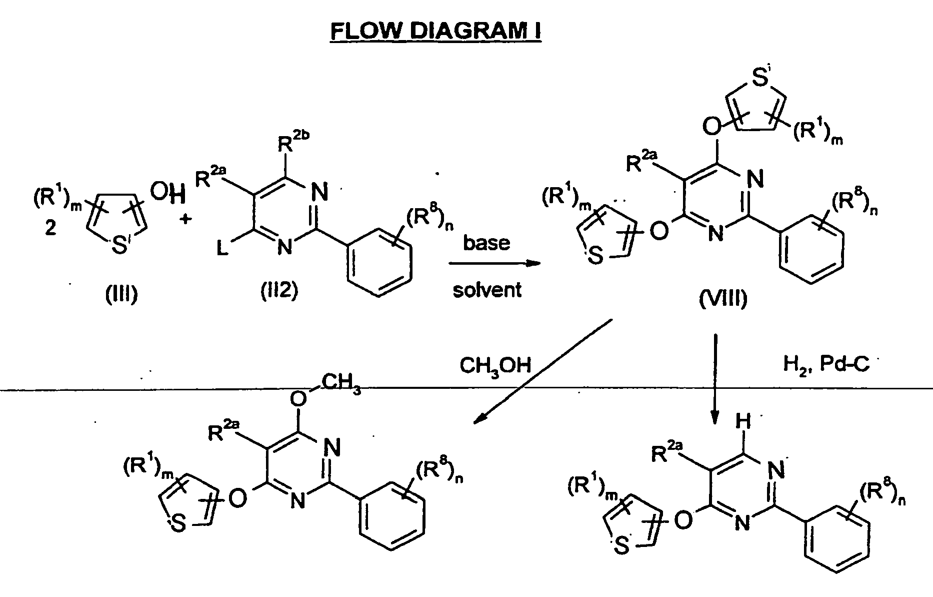

Preferred formula VIII compounds of the invention are those compounds wherein R² is hydrogen.

More preferred compounds of formula VIII are those compounds having the structure of formula VIIIA 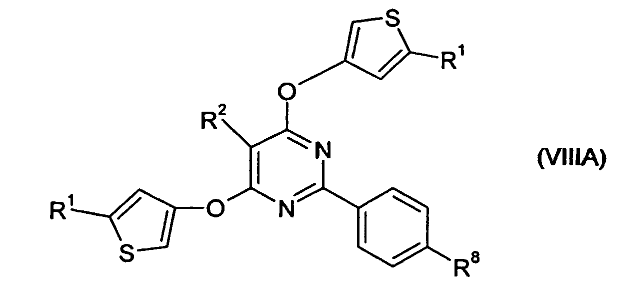 wherein R¹ and R⁸ are as described for formula 12.

Particularly preferred are those compounds wherein R¹ is CF₃ or CH₃SO₂; R² is hydrogen; R⁸ is CF₃; and R² is hydrogen.

The present invention relates to the novel compounds of formula VIII.

Compounds of formula III may be prepared according to conventional procedures such as the conjugative addition of the appropriately substituted butenoate of formula XI with a thioglycolate of formula XII in the presence of a base to form a thienyl--2-carboxylate compound of formula XIII. The formula XIII compound may then be hydrolyzed to the corresponding carboxylic acid of formula XIV, which may then be decarboxylated to form the desired formula III compound wherein the hydroxyl group is attached in the 3 position. The reaction sequence is shown in flow diagram II.

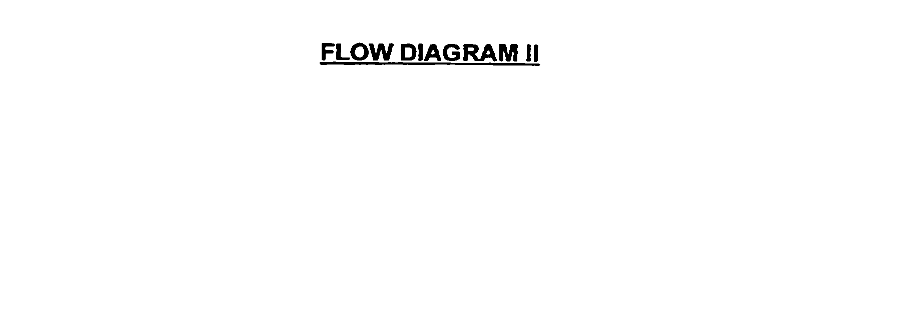

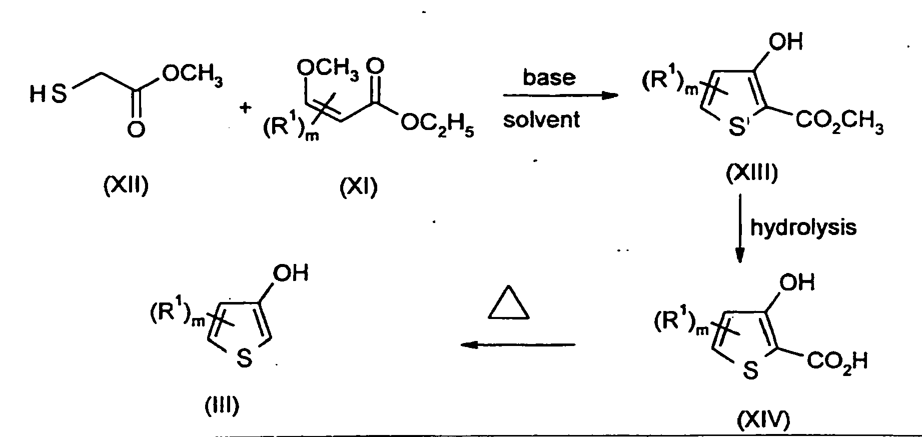

The present invention provides a compound of formula IIIA 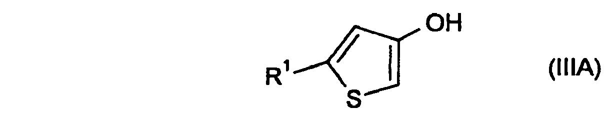 wherein R¹ has the meaning given hereinabove.
Compounds of formula IIIA are preferred,
wherein R¹ is CN, R³SOₚ,
C₁-C₆alkyl optionally substituted with one or more halogen, C₁-C₄-alkoxy, C₁-C₄haloalkoxy, C₁-C₄alkoxycarbonyl, phenyl optionally substituted with one or more halogen, C₁-C₄alkyl or C₁-C₄haloalkyl groups, OH, CN, NO₂, or C₁-C₄-alkylsulfonyl groups, C₁-C₄alkoxy optionally substituted with one or more halogen, OH, CN, NO₂ groups,
R³ is C₁-C₄alkyl or C₁-C₄haloalkyl;
p is 0 or an integer of 1 or 2.

Preferred compounds of formula IIIA are those compounds wherein R¹ is C₁-C₄haloalkyl or C₁-C₄haloalkylsulfonyl and more preferably wherein R¹ is CF₃ or CF₃SO₂.

Compounds of formula IIIA are particularly useful for preparing many of the herbicidal compounds of formula IA when employed in the processes illustrated hereinabove.

Furthermore, the invention relates to a process for the preparation of the compounds of formula IIIA, in which R¹ is as defined hereinabove for formula I, which comprises the steps of
(a) saponification of a compound of formula XIII 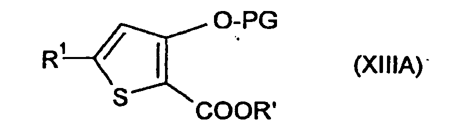 in which R¹ is as described for formula IIIA; R¹ is C₁₋₆ alkyl; and PG represents a hydrogen atom or a protecting group, to obtain a compound of formula XIVA 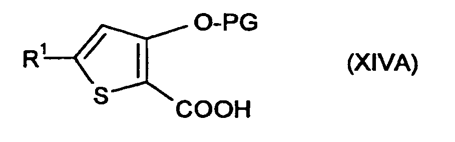
(b) decarboxylation of the compound of formula XIVA; and
(c) optionally cleaving of the protecting group.

PG represents a protecting group, in particular a benzyl group. The deprotection method depends on the protecting group used. When the protecting group represents a benzyl group, treatment with iodotrimethylsilane in tetrachloromethane is preferred.

The reactions according to (A), (B), (C), (D), (E) may be carried out in the absence or presence of a solvent which promotes the reaction or at least does not interfere with it. Preferred are polar, aprotic or protic solvents, suitably being N,N-dimethylformamide, dimethylsulfoxide, sulfolane, acetonitrile, methyl ethyl ketone, or an ether, such as tetrahydrofurane or dioxane, or alcoholes, or water, or mixtures thereof.

The reactions are carried out at a temperature between ambient temperature and the reflux temperature of the reaction mixture, preferably at elevated temperature, especially at reflux temperature. Conveniently substantially equimolar amounts of reactants are used. It may be expedient, however, to use one reactant in excess.

The reactions may be carried out in the presence of a basic compound such as an alkali hydroxide, bicarbonate or carbonate, e. g. sodium or potassium hydroxide, bicarbonate or carbonate, an alkali alkoxide, e. g. sodium ethoxide, or an organic base such as triethylamine.

A hydroxy compound used in the above reactions may be present in form of a salt, preferably as a salt of an alkali metal, particularly of sodium or potassium. The presence of a copper salt may be suitable.

Suitable leaving groups L are e.g. alkyl- and arylsulfonyl, alkyl- and arylsulfonyloxy, perfluoroalkylsulfonyloxy, nitro groups and halogen atoms, particularly fluorine, chlorine and bromine atoms.

The metal salts are conveniently generated by reaction of the hydroxythiophene of formula III or B -Z- H compounds of formula IV, the alcohols or thiols with a suitable metal base, a metal carbonate or hydride.

The prepared compounds of formula I may be isolated and purified using conventional methods and techniques.

The starting compounds for the preparation of compounds of this invention can be prepared according to known methods.

For compounds of formula II, IV, or VI certain substituents R² like alkyl, alkoxy, alkylthio, alkylamino, dialkylamino, amino or halo, can be introduced onto the pyridine ring by displacement of a alkyl- or arylsulfonyl, alkyl- or arylsulfonyloxy, nitro, or halogen group. Halogen atoms may also be introduced by diazotization of an amino group. The diazotization reaction may be carried out in an aqueous medium and the diazonium compound can be reacted e. g. with CuCl, CuBr, CuCN or KI to introduce the chlorine, bromine, iodine atom or the cyano group.

The compounds used as starting material are partly known and partly novel. The intermediate compounds of formula VI are known for example from GB 2 285 045. Intermediates of formula II are known from WO 94/22833, EP 0 447 004, EP 0 572 093, EP 0 693 490, EP 0 692 474, EP 0 694 538, EP 0 707 001, EP 0 723 960, and GB 2 285 045.

The present invention also provides the use of the compounds of formula I as herbicides. Further, in accordance with the invention there is provided a method of combating undesired plant growth at a locus by treating the locus with a composition according to the invention or an effective amount of a compound of formula I. As a useful action is by foliar spray application, the locus is most suitably the plants in a crop area, typical crops being cereals, maize, soya bean, sunflower or cotton. However, application may also be to the soil for those compounds having pre-emergence herbicidal action, or to the water of paddy rice fields. The dosage of active ingredient used may, for example be in the range of from 0.005 to 3 kg/ha, preferably 0.01 to 1 kg/ha.

The compounds of general formula I have been found to have herbicidal activity. Accordingly, the invention further provides a herbicidal composition which comprises an active ingredient, which is at least one compound of formula I as defined above, and one or more carriers. A method of making such a composition is also provided which comprises bringing a compound of formula I as defined above into association with the carrier(s). Such a composition may contain a single active ingredient or a mixture of several active ingredients of the present invention. It is also envisaged that different isomers or mixtures of isomers may have different levels or spectra of activity and thus compositions may comprise individual isomers or mixtures of isomers.

A composition according to the invention preferably contains from 0.5% to 95% by weight (w/w) of active ingredient.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including material which is normally a gas but which has been compressed to form a liquid.

The compositions may be manufactured into e.g. emulsion concentrates, solutions, oil in water emulsions, wettable powders, soluble powders, suspension concentrates, dusts, granules, water dispersible granules, micro-capsules, gels and other formulation types by well-established procedures. These procedures include intensive mixing and/or milling of the active ingredients with other substances, such as fillers, solvents, solid carriers, surface active compounds (surfactants), and optionally solid and/or liquid auxilaries and/or adjuvants. The form of application such as spraying, atomizing, dispersing or pouring may be chosen like the compositions according to the desired objectives and the given circumstances.

Solvents may be aromatic hydrocarbons, e.g. Solvesso^{®} 200, substituted naphthalenes, phthalic acid esters, such as dibutyl or dioctyl phthalate, aliphatic hydrocarbons, e.g. cyclohexane or paraffins, alcohols and glycols as well as their ethers and esters, e.g. ethanol, ethyleneglycol mono- and dimethyl ether, ketones such as cyclohexanone, strongly polar solvents such as N-methyl-2-pyrrolidone, or γ-butyrolactone, higher alkyl pyrrolidones, e.g. n-octylpyrrolidone or cyclohexylpyrrolidone, epoxidized plant oil esters, e.g. methylated coconut or soybean oil ester and water. Mixtures of different liquids are often suitable.

Solid carriers, which may be used for dusts, wettable powders, water dispersible granules, or granules, may be mineral fillers, such as calcite, talc, kaolin, montmorillonite or attapulgite. The physical properties may be improved by addition of highly dispersed silica gel or polymers. Carriers for granules may be porous material, e.g. pumice, kaolin, sepiolite, bentonite; non-sorptive carriers may be calcite or sand. Additionally, a multitude of pre-granulated inorganic or organic materials may be used, such as dolomite or crushed plant residues.

Pesticidal compositions are often formulated and transported in a concentrated form which is subsequently diluted by the user before application. The presence of small amounts of a carrier which is a surfactant facilitates this process of dilution. Thus, preferably at least one carrier in a composition according to the invention is a surfactant. For example, the composition may contain at two or more carriers, at least one of which is a surfactant.

Surfactants may be nonionic, anionic, cationic or zwitterionic substances with good dispersing, emulsifying and wetting properties depending on the nature of the compound according to general formula I to be formulated. Surfactants may also mean mixtures of individual surfactants.

The compositions of the invention may for example be formulated as wettable powders, water dispersible granules, dusts, granules, solutions, emulsifiable concentrates, emulsions, suspension concentrates and aerosols. Wettable powders usually contain 5 to 90% w/w of active ingredient and usually contain in addition to solid inert carrier, 3 to 10% w/w of dispersing and wetting agents and, where necessary, 0 to 10% w/w of stabilizer(s) and/or other additives such as penetrants or stickers. Dusts are usually formulated as a dust concentrate having a similar composition to that of a wettable powder but without a dispersant, and may be diluted in the field with further solid carrier to give a composition usually containing 0.5 to 10% w/w of active ingredient. Water dispersible granules and granules are usually prepared to have a size between 0.15 mm and 2.0 mm and may be manufactured by a variety of techniques. Generally, these types of granules will contain 0.5 to 90% w/w active ingredient and 0 to 20% w/w of additives such as stabilizer, surfactants, slow release modifiers and binding agents. The so-called "dry flowables" consist of relatively small granules having a relatively high concentration of active ingredient. Emulsifiable concentrates usually contain, in addition to a solvent or a mixture of solvents, 1 to 80% w/v active ingredient, 2 to 20% w/v emulsifiers and 0 to 20% w/v of other additives such as stabilizers, penetrants and corrosion inhibitors. Suspension concentrates are usually milled so as to obtain a stable, non-sedimenting flowable product and usually contain 5 to 75% w/v active ingredient, 0.5 to 15% w/v of dispersing agents, 0.1 to 10% w/v of suspending agents such as protective colloids and thixotropic agents, 0 to 10% w/v of other additives such as defoamers, corrosion inhibitors, stabilizers, penetrants and stickers, and water or an organic liquid in which the active ingredient is substantially insoluble; certain organic solids or inorganic salts may be present dissolved in the formulation to assist in preventing sedimentation and crystalization or as antifreeze agents for water.

Aqueous dispersions and emulsions, for example compositions obtained by diluting the formulated product according to the invention with water, also lie within the scope of the invention.

Of particular interest in enhancing the duration of the protective activity of the compounds of this invention is the use of a carrier which will provide slow release of the pesticidal compounds into the environment of a plant which is to be protected as disclosed for example by U.S patent no. 5,705,174.

The biological activity of the active ingredient can also be increased by including an adjuvant in the spray dilution. An adjuvant is defined here as a substance which can increase the biological activity of an active ingredient but is not itself significantly biologically active. The adjuvant can either be included in the formulation as a coformulant or carrier, or can be added to the spray tank together with the formulation containing the active ingredient.

As a commodity the compositions may preferably be in a concentrated form whereas the end user generally employs diluted compositions. The compositions may be diluted to a concentration down to 0.001% of active ingredient. The doses usually are in the range from 0.01 to 10 kg a.i./ha.

Examples of formulations according to the invention are:

| Emulsion Concentrate (EC) | | |
|---|---|---|
| Active Ingredient | Compound of Example 11 | 30 % (w/v) |
| Emulsifier(s) | Atlox^{®} 4856 B / Atlox^{®} 4858 B ¹⁾ | 5 % (w/v) |
| | (mixture containing calcium alkyl aryl | |
| | sulfonate, fatty alcohol ethoxylates and light | |
| | aromatics / mixture containing calcium alkyl | |
| | aryl sulfonate, fatty alcohol ethoxylates and | |
| | light aromatics) | |
| Solvent | Shellsol^{®} A ²⁾ | to 1000 ml |
| | (mixture of C₉ - C₁₀ aromatic hydrocarbons) | |

| Suspension Concentrate (SC) | | |
|---|---|---|
| Active Ingredient | Compound of Example 11 | 50 % (w/v) |
| Dispersing agent | Soprophor^{®} FL ³⁾ | 3 % (w/v) |
| | (polyoxyethylene polyaryl phenyl ether | |
| | phosphate amine salt) | |
| Antifoaming agent | Rhodorsil^{®} 422 ³⁾ | 0.2 % |
| | (nonionic aqueous emulsion of | (w/v) |
| | polydimethylsiloxanes) | |
| Structure agent | Kelzan^{®} S ⁴⁾ | 0.2 % |
| | (Xanthan gum) | (w/v) |
| Antifreezing agent | Propylene glycol | 5 % (w/v) |
| Biocidal agent | Proxel^{® 5)} | 0.1 % |
| | (aqueous dipropylene glycol solution | (w/v) |
| | containing 20% 1,2-benisothiazolin-3-one) | |
| Water | | to 1000 ml |

| Wettable Powder (WP) | | |
|---|---|---|
| Active Ingredient | Compound of Example 12 | 60 % (w/w) |
| Wetting agent | Atlox^{®} 4995 ¹⁾ | 2 % (w/w) |
| | (polyoxyethylene alkyl ether) | |
| Dispersing agent | Witcosperse^{®} D-60 ⁶⁾ | 3 % (w/w) |
| | (mixture of sodium salts of condensed | |
| | naphthalene sulfonic acid and | |
| | alkylarylpolyoxy acetates | |
| Carrier / Filler | Kaolin | 35 % (w/w) |

| Water Dispersible Granules (WG) | | |
|---|---|---|
| Active Ingredient | Compound of Example 12 | 50 % (w/w) |
| Dispersing / | Witcosperse^{®} D-450 ⁶⁾ | 8 % (w/w) |
| Binding agent | (mixture of sodium salts of condensed | |
| | naphthalene sulfonic acid and alkyl | |
| | sulfonates) | |
| Wetting agent | Morwet^{®} EFW ⁶⁾ | 2 % (w/w) |
| | (formaldehyde condensation product) | |
| Antifoaming agent | Rhodorsil^{®} EP 6703 ³⁾ | 1 % (w/w) |
| | (encapsulated silicone) | |
| Disintegrant | Agrimer^{®} ATF⁷⁾ | 2 % (w/w) |
| | (cross-linked homopolymer of N-vinyl-2- | |
| | pyrrolidone) | |
| Carrier / Filler | Kaolin | 35 % (w/w) |

| | | |
|---|---|---|
| ¹⁾ available from ICI Surfactants ²⁾ available from Deutsche Shell AG ³⁾ available from Rhodia (former Rhône-Poulenc) ⁴⁾ available from Kelco Co. ⁵⁾ available from Zeneca ⁶⁾ available from Witco ⁷⁾ available from International Speciality Products | | |

The compositions of this invention can also comprise other compounds having biological activity, e.g. compounds having similar or complementary pesticidal activity or compounds having plant growth regulating, fungicidal or insecticidal activity. These mixtures of pesticides can have a broader spectrum of activity than the compound of general formula I alone. Furthermore, the other pesticide can have a synergistic effect on the pesticidal activity of the compound of general formula I.

The active ingredients according to the invention can be employed alone or as formulations in combination with conventional herbicides. Such combinations of at least two herbicides can be included in the formulation or also added in a suitable form with the preparation of the tank mix. For such mixtures at least one of the following known herbicides can be used:
amethydione, metabenzthiazuron, metamitron, metribuzin, 2,4-D, 2,4-DB, 2,4-DP, alachlor, alloxydim, asulam, atrazin, bensulfuron, bentazon, bifenox, bromoxynil, butachlor, chloridazon, chlorimuron, chlorpropham, chlorsulfuron, chlortoluron, cinmethylin, clopyralid, cyanazin, cycloate, cycloxydim, dichlobenil, diclofop, eptame, ethiozin, fenoxaprop, fluazifop, fluometuron, fluridone, fluroxypyr, fomesafen, glyphosate, haloxyfop, hexazinone, imazamethabenz, imazapyr, imazaquin, imazethapyr, ioxynil, isoproturon, lactofen, MCPA, MCPP, mefenacet, metazachlor, metolachlor, metsulfuron, molinate, norflurazon, oryzalin, oxyfluorfen, pendimethalin, picloram, pretilachlor, propachlor, pyridate, quizalofopethyl, sethoxydim, simetryne, terbutryne, thiobencarb, triallate, trifluralin, diflufenican, propanil, triclopyr, dicamba, desmedipham, acetochlor, fluoroglycofen, halosafen, tralkoxydim, amidosulfuron, cinosulfuron, nicosulfuron, pyrazosulfuron, oxasulfuron, azimsulfuron, thiameturon, thifensulfuron, triasulfuron, tribenuron, esprocarb, prosulfocarb, terbutylazin, benfuresate, clomazone, di-methazone, dithiopyr, isoxaben, quinchlorac, qinmerac, sulfosate, cyclosulfamuron, imazamox, imazamethapyr, flamprop-M-methyl, flamprop-M-isopropyl, picolinafen, thiafluamide, isoxaflutole, flurtamone, daimuron, bromobutide, methyldimron, dimethenamid, sulcotrione, sulfentrazone, oxadiargyl, acifluorfen, cafenstrole, carfentrazone, diuron, glufosinate.

Mixtures with other active ingredients like fungicides, insecticides, acaricides and nematicides are possible.

For a more clear understanding of the invention, specific examples are set forth below. These examples are merely illustrations and are not to be understood as limiting the scope and underlying principles of the invention in any way. Various modifications of the invention in addition to those shown and described herein will become apparent to those skilled in the art from the following examples and foregoing description. Such modifications are also intended to fall within the scope of the appended claims.

The structures of the compounds prepared in the following examples were additionally confirmed by NMR and mass spectrometry.

### Example 1: Preparation of 3-hydroy-5-trifluoromethylthiophene

### Method A

### 1A Ethyl 3-methoxy-3-trifluoromethylacrylate

Cesium carbonate (132.8 g) was added to a mixture of ethyl 4,4,4-trifluoroacetoacetate (75.0 g) and dimethylformamide (400 ml). The reaction mixture was heated to 70 °C for 30 minutes. A mixture of methyl tosylate (83.4 g) and dimethylformamide (150 ml) was added to the resulting reaction mixture within 40 minutes. The mixture was heated for 3 hours and cooled to room temperature. Upon dilution with water (800 ml) the reaction mixture was extracted with diethyl ether three times. The combined organic phases were washed with water and dried. The mixture was concentrated and the residue distilled under reduced pressure to yield the product as a clear liquid (48.5 g, 60 %) with a boiling point of 62-70 °C at 12 mm.

### 1B Methyl (3-hydroxy-5-trifluoromethylthien-2-yl)-carboxylate

A solution of 1 M potassium hydroxide in methanol (30 ml) is added to a cooled mixture of 1A (4.6 g), methyl thioglycolate (2.46 g) and methanol (10 ml). The resulting reaction mixture was stirred for 24 hours at room temperature. Then the mixture was poured on ice and acidified with 6N sulfuric acid (pH = 2). The mixture is extracted with diethyl ether twice. The combined organic phases were washed with water and dried. The mixture was concentrated and the residue is distilled under reduced pressure to yield the product as a clear liquid (3.4 g, 65 %) with a boiling point of 42-45 °C at 0.10 mm.

### 1C (3-Hydroxy-5-trifluoromethylthien-2-yl)-carboxylic acid

A mixture of 1B (2.38 g) and methanol (20 ml) was added to a stirred solution of sodium hydroxide (1.68 g) in water (20 ml). The reaction mixture was heated at reflux for 3 hours. The reaction mixture was cooled to room temperature and concentrated in vacuo. The concentrate was cooled to 5 °C and acidified with concentrated HCl (3.5 ml). The resulting suspension was stirred at 5 °C for 30 minutes. The solid was collected by filtration, washed with water, then dried in vacuo at 35-40 °C to give the free acid (1.45 g, 65 %).

### 1D 3-Hydroxy-5-trifluoromethylthiophen

1C (1.80 g) was slowly heated under argon. Evolution of gas was observed at 90 °C. Heating was continued for additional 3.5 hours at 90°C. The resulting oil was distilled under reduced pressure (boiling point 70-74 °C at 4 mm) to yield 1.18 g (82 %) of compound 1D.

### Method B

### 1E Methyl (3-benzyloxy-5-trifluoromethylthien-2-yl)-carboxylate

A mixture of 1B (5.0 g) and dimethylformamide (50 ml) is treated with sodium hydride (1.06 g). Benzylbromide (3.15 ml) was slowly added to the resulting reaction mixture and stirred at room temperature for 20 hours. The reaction mixture was poured into water. The mixture was extracted with diethyl ether twice. The combined organic phases were washed with water, dried and concentrated in vacuo. The crude product was chromatographed (hexane / dichloromethane, 1/1) to give the product as a white solid (4.5 g, 64 %) with a melting point of 52-53.5 °C.

### 1 F (3-Benzyloxy-5-trifluoromethylthien-2-yl)-carboxlic acid

A mixture of 1E (3.80 g) and tetrahydrofuran (12 ml) was heated to reflux in 2N sodium hydroxide (12 ml) for 12 hours. Then the mixture was poured on ice and acidified with 6N sulfuric acid (pH = 1-2). The mixture is extracted with diethyl ether twice. The combined organic phases were washed with water and dried. The mixture was concentrated and the residue is distilled under reduced pressure to yield the product as a white solid (3.22 g, 89 %) with a melting point of 142-144 °C.

### 1G 3-Benzyloxy-5-trifluoromethylthionhen

A mixture of 1F (14.5 g) and quinoline (50 ml) was treated with copper powder (4.57 g) and heated to 150 °C. The reaction mixture is heated for 25 minutes at 150 °C and cooled to room temperature. The mixture was filtered and washed with water. Aqueous quinoline was acidified with 6N HCl (pH = 2) and extracted with diethyl ether twice. The combined organic phases were washed with water and dried. The mixture was concentrated and the residue was chromatographed to yield a yellow liquid (8.74 g, 71 %).

### 1D 3-Hydroxy-5-trifluoromethylthiophene

A mixture of 1G (7.75 g) and tetrachloromethane (50 ml) was treated with iodotrimethylsilane (12.30 ml) and heated to 60 °C for 12 hours. The reaction mixture was stirred at room temperature for 12 hours. Water (50 ml) was added and the resulting reaction mixture was extracted with dichloromethane three times. The combined organic phases were washed with water and dried. The crude reaction mixture was eluted through hexane (100 g/silica gel) to remove benzyliodide and then with diethyl ether. The etheral phases were concentrated and distilled in vacuo to give the product (3.33 g, 74 %) having a boiling point of 65-66 °C at 4 mm. Analogously is obtainned 4-hydroxy-2-(methylsulfonyl)thiophene, mp 109-111 °C.

### Example 2: Preparation of 2-(3,4-difluorobenzyloxy)-6-(5-trifluoromethylthienyl-3-oxy)-4-methylpyridine (not claimed)

To 0.34g potassium *tert-*butylate (3 mmol) in 10 ml dry diglyme are added 0.51g (3 mmol) 1D. After stirring at room temperature for 30 min., 0.55g (2 mmol) 2-bromo-4-methyl-6-(3,4-difluorobenzyloxy)-pyridine (prepared analogously to the method disclosed in WO 94/22833), 0.3g CuBr and a catalytic amount of 18-crown-6 ether are added. The mixture is heated to 130°C under nitrogen atmosphere for 16 hrs. Another 0.51g hydroxythiophene and 0.34 g potassium *tert*-butylate are added and heating is continued for 16hrs. The mixture is diluted with toluene, washed with water and purified by flash chromatography.
Yield 0.35 g oil

### Example 3: Preparation of 3,5-difluoro-2-(4-fluorobenzyloxy)-4-methyl-6-(5-trifluoromethylthienyl-3-oxy)-pyridine (not claimed)

### 3A 4-methyl-2,3,5-trifluoro-6-(5-trifluoromethylthienyl-3-oxy)-pyridine

A mixture of 4-methyl-2,3,5,6-tetrafluoropyridine (20 mmol, 3.3 g), sodium hydride (23 mmol) and 1D (22 mmol, 3.7 g) in anhydrous acetonitrile (100 ml) is stirred at room temperature for 5 hours. The solvent is removed under reduced pressure. Pentane/ethyl acetate (1/1 by volume) is added to the residue and the mixture is washed twice with 2N sodium hydroxide and 5 times with water. After drying of the organic layer, the solvent is removed and the product is purified by flash chromatography and distillation under reduced pressure: One obtains the product as an oil (2.75 g, b.p. 110°C at 0.0015 mbar).

### 38 3,5-Difluoro-6-(4-fluorophenoxy)-4-methyl-2-(5-trifluoromethylthienyl-3-oxy)-pyridine

A mixture of 3A (0.85 g, 2.7 mmol), 60% sodium hydride (0.13 g, 3.3 mmol) and 4-fluorobenzylalcohol (3,0 mmol) in anhydrous sulfolane (3 ml) is heated to 85°C for 5 hours. The reaction mixture is diluted with pentane/ethyl acetate (1/1 by volume) and filtered through a bed of silica gel. The filtrate is washed 5 times with water, the organic layer is dried with anhydrous magnesium sulfate, and the solvents are removed in vacuo. The residue is purified by a flash silica gel chromatography using pentane/toluene in a ratio of 4/1. 0.6 g (53% yield) of 4B are obtained as a colourless oil.

### Examples 5 - 6:

Further Examples are prepared according to the general method of Examples 2 to 3 and are listed in Table 1.

**Table 1**

| | | | | | | |
|---|---|---|---|---|---|---|
| 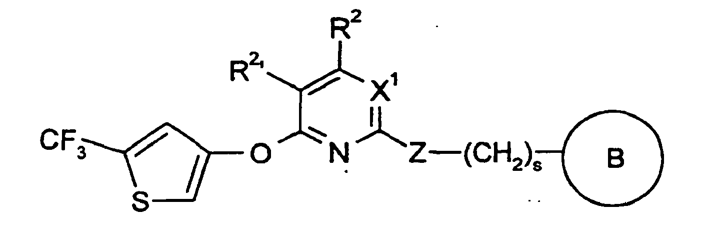 | | | | | | |

| Ex. No. | R² | B | R^{2'} | X¹ | Z | s |
|---|---|---|---|---|---|---|
| 5 | CH₃O | 5-CF₃-thien-3-yl | H | N | O | 0 |
| 6 | CH₃O | phenyl | H | N | O | 1 |

### EXAMPLE 7

### 7A Preparation of p-(Trifluoromethyl)benzamidine Hydrochloride

A stirred solution of *p*-(trifluoromethyl)benzonitrile (100 g, 0.585 mol) in toluene is treated with NaNH₂ (38 g, 0.878 mol, 90% pure) and dibenzo-18-crown-6 (1.5 g), heated at reflux temperatures for 4 hours, cooled to 10°C and treated slowly with 175 ml of concentrated HCl over a 30 minute period. The resultant reaction mixture is filtered and the filtercake is washed with toluene and dried to give a powdery yellowish solid. The solid is dissolved in ethanol and filtered. The filtrate is concentrated *in vacuo* to give a yellow solid residue. The residue is slurried in ethyl acetate and filtered. The filtercake is washed with ether and dried to give the title product as a yellow solid, 132.3 g (quantitative yield).

### 7B 6-Hydroxy-5-methyl-2-α,α,α-trifluoro-p-tolyl)pyrimidine

A mixture of 7A (31.7 g, 0.141 mol) and a 25% NaOCH₃ solution in methanol (60.9 g, 0.282 mol NaOCH₃) is stirred for 10 minutes, treated with methyl 2-formylpropionate (14.9 g, 0.128 mol) and stirred at ambient temperatures until reaction is complete by HPLC. The resultant reaction mixture is concentrated *in vacuo* to give a solid residue. The residue is treated with 1N NaOH, acidified with 10% HCl to pH 4 and filtered. The filtercake is washed with water and dried *in vacuo* to give the title product as a white solid, 30.35 g (85% yield), mp 273-275°C.

### 7C Substituted 6-hydroxy-2-(α,α,α-trifluoro-o-tolyl)pyrimidine

Using essentially the same procedure described in Example 7B and substituting the appropriate acetate or malonate reagent, the following substituted 2-hydroxypyrimidines are obtained.

**Table 2**

| | | | | |
|---|---|---|---|---|
| 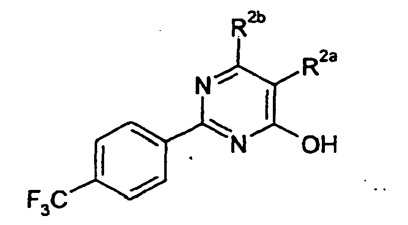 | | | | |

| Reagent | R^{2b} | R^{2a} | mp °C | yield |
|---|---|---|---|---|
| | | | | (%) |
| methyl propionylacetate | C₂H₅ | H | 181 | 86 |
| ethyl butyrylacetate | n-C₃H₇ | H | 138-141 | 59 |
| ethyl acetoacetate | CH₃ | H | 209 | 93 |
| methyl 4-methoxyacetoacetate | CH₂OCH₃ | H | 180-182 | 82 |
| dimethyl ethylmalonate | OH | C₂H₅ | >270 | 64 |
| dimethyl malonate | OH | H | >275 | 56 |

### 7D 6-Chloro-5-methyl-2-(α,α,α-trifluoro-p-tolyl)pyrimidine

A mixture of 7B (5.00 g, 18.7 mmol) and 10 ml of POCl₃ is heated at reflux temperatures for 2 hours, allowed to cool to room temperature overnight and concentrated *in vacuo* to a thick paste residue. The residue is diluted with water and extracted with ethyl acetate. The ethyl acetate extracts are combined, dried with brine and concentrated *in vacuo* to afford the title product as a tan solid, 2.72 g (51% yield) mp 110-112°C.

### 7E Substituted 6-chloro-2-(α,α,α-trifluoro-p-tolyl)pyrimidines

Using essentially the same procedure described in Example 7D and employing the appropriately substituted 6-hydroxypyrimidine, the following compounds are obtained.

**Table 3**

| | | | |
|---|---|---|---|
| 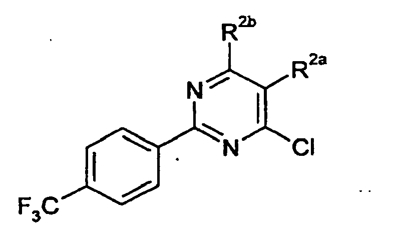 | | | |

| R^{2b} | R^{2a} | mp (°C) | yield (%) |
|---|---|---|---|
| C₂H₅ | H | 30-32 | 85 |
| n-C₃H₇ | H | oil | 80 |
| CH₃ | H | 62 | 91 |
| Cl | H | oil | 73 |
| Cl | C₂H₅ | 77-78 | 51 |
| CH₂OCH₃ | H | oil | 81 |

### EXAMPLE 8: Preparation of 4-Methyl-2-(α,α,α-trifluoro-p-tolyl)-6-{[5-(trifluoromethyl)-3-thienyl]oxy}pyrimidine

A mixture of 1D (0.50 g, 2.98 mmol) and 7D (0.31g, 2.98 mmol) in dimethylformamide is treated with K₂CO₃ (0.62 g, 4.47 mmol), heated at 100°C for 17 hours, cooled to room temperature and diluted with water. The resultant reaction mixture is extracted with ether. The ether extracts are combined, washed sequentially with water and brine, dried over MgSO₄ and concentrated *in vacuo* to give a brown solid residue. The residue is chromatographed (silica gel, ethyl acetate/hexanes, 20/80) to give the title product as a light yellow solid, 0.88 g (73% yield), mp 83.5-85°C.

### EXAMPLE 9 - 22: Preparation of Substituted 6-(Thienyloxy)-pyrimidines

Using essentially the same procedure described in Example 8 and employing the appropriate 6-chloro-pyrimidine and 3-hydroxythiophene reactants the following compounds are obtained.

**Table 4**

| | | | | | |
|---|---|---|---|---|---|
| 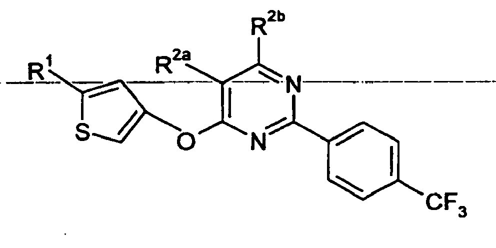 | | | | | |

| Example | R | R^{2a} | R^{2b} | mp (°C) | yield (%) |
|---|---|---|---|---|---|
| Number | | | | | |
| 9 | CH₃SO₂ | H | CH₃ | 145-146 | 35 |
| 10 | CF₃ | CH₃ | H | 85-86 | 72 |
| 11 | CH₃SO₂ | CH₃ | H | 156-158 | 57 |
| 12 | CF₃ | H | C₂H₅ | 61-63 | 65 |
| 13 | CF₃ | H | n-C₃H₇ | 53-54.5 | 52 |
| 14 | CF₃ | C₂H₅ | Cl | 82.5-85 | 72 |
| 15 | CF₃ | H | Cl | 85-86 | 75 |
| 16 | CF₃ | H | CH₂OCH₃ | oil | 58 |
| 17 | CO₂CH₃ | H | CH₃ | 120-122 | 52 |
| 18 | CH₂OH | H | CH₃ | 106-108 | 62 |
| 19 | CHO | H | CH₃ | 152-153.5 | 89 |
| 20 | CO₂H | H | CH₃ | 203-204 | 86 |
| 21 | CHF₂ | H | CH₃ | 57-58 | 54 |
| 22 | CF₃ | H | H | 61-63 | 53 |

### EXAMPLE 23

### 23A 2-(α,α,α-Trifluoro-p-tolyl)-4,6-di{[5-(trifluoromethyl)-3-thienyl]oxy}pyrimidine

A mixture of 4,6-dichloro-2-(α,α,α-trifluoro-p-tolyl)pyrimidine (2.15 g, 7.34 mmol) and 1D (2.47 g, 14.7 mmol) in dimethylformamide is treated with K₂CO₃ (2.52 g, 18.3 mmol), heated at 100°C for 3 hours, cooled to room temperature, poured onto water and neutralized with 6N HCL to pH 6-7. The resultant reaction mixture is extracted with ether. The ether extracts are washed sequentially with water and brine, dried over MgSO₄ and concentrated *in vacuo* to give a yellow solid residue. The residue is purified by flash chromatography (silica gel, methylene chloride) to afford the title product as an off-white solid, 3.03 g (74%), mp 93-95°C.

### 23B 4-Methoxy-2-(α,α,α,-trifluoro-p-tolyl)-6-{[5-(trifluoromethyl)-3-thienyl]oxy}pyrimidine

A solution of 23A(1.50 g, 2.70 mmol) in dimethylformamide is treated with 25% NaOCH₃ in methanol (0.58 g, 2.70 mmol), heated to 60°C for 1.5 hours, cooled to room temperature, poured onto water and acidified to pH 5 with 6N HCl. The resultant reaction mixture is extracted with ether. The ether extracts are combined, washed sequentially with water and brine, dried over MgSO₄ and concentrated *in vacuo* to give a solid residue. The residue is chromatographed (silica gel, ethyl acetate/hexanes, 3/97) to afford the title product as a white solid, 0.58 g (51% yield), mp 91-93°C.

### EXAMPLE 24: Preparation of 5-Ethyl-2-(α,α,α-trifluoro-p-tolyl)-6-{[5-(trifluoromethyl)-3-thienyl]oxy}pyrimidine

A solution of 4-chloro-5-ethyl-2-(α,α,α-trifluoro-*p*-tolyl)-6-{[5-(trifluoromethyl)-3-thienyl]oxy}pyrimidine (1.06 g, 2.34 mmol) in a 1:1 mixture of methanol:ethyl acetate is treated with triethylamine (0.80 ml, 5.86 mmol) and 10% Pd-C (0.32 g, 30 wt%). The resultant mixture is placed on a PARR hydrogenator at 50 psi for 2 hours (reaction is complete by TLC). The resultant mixture is filtered. The filtrate is concentrated. The concentrate is dissolved in ethyl acetate/chloroform and chromatographed (silica gel, methylene chloride/hexanes, 20/80) to give the title product as a white solid, 0.69 g (70%), mp 67-68°C.

### Herbicidal Activity

To evaluate their herbicidal activity, compounds according to the invention are tested using a representative range of plants:

### Plant Species Used

| | | |
|---|---|---|
| ABUTH | *Abutilon theophrasti* | velvetleaf |
| ALOMY | *Alopecurus myosuroides* | blackgrass |
| AMBEL | *Ambrosia artemisiifolia, L* | ragweed |
| CASOB | *Senna obtusifolia* | sicklepod |
| CHEAL | *Chenopodium album* | lambsquarters |
| DIGSA | *Digitaria sanguinalis* | crabgrass |
| ECHCG | *Echinichloa crus-galli* | bamyardgrass |
| GALAP | *Galium aparine* | cleaver |
| GLXMA | *Glycine max* | soybeans |
| IPOHE | *Ipomoea hederacea* | morning glory |
| MATIN | *Matricaria inodora* | mayweed |
| PANDI | *Panicum* | Panicum |
| | *dichotomiflorum* | |
| SETVI | *Setaria viridis* | green foxtail |
| TRZAW | *Triticum aestivum* | winter wheat |
| ZEAMX | *Zea mays* | maize |

The pre-emergence tests involve spraying a liquid formulation of the compound onto the soil in which the seeds of the plant species mentioned above has recently been sown. The soil used in the tests is a sandy loam. The formulations used in the tests are prepared from solutions of the test compounds in acetone containing 0.4% by weight of an alkylphenol/ethylene oxide condensate available under the trade mark TRITON X-155. These acetone solutions are diluted with water and the resulting formulations applied at dosage levels corresponding to dose rates between 0.4 kg and 0.0125 kg, of active material per hectare in a volume equivalent to 900 litres per hectare. In these tests untreated sown soil are used as controls.

The herbicidal effects of the test compounds are assessed visually twenty-one days after spraying the foliage and the soil and visual ratings are made using the rating scale shown below.

| Rating Scale | |
|---|---|
| | % Control |
| Rating | (As Compared To Check) |
| 9 | 100 |
| 8 | 91-99 |
| 7 | 80-90 |
| 6 | 65-79 |
| 5 | 45-64 |
| 4 | 30-44 |
| 3 | 16-29 |
| 2 | 6-15 |
| 1 | 1-5 |
| 0 | No Effect |

The results of the pre-emergence assessment are set out in Tables 5 to 6.

**Table 5**

| Pre-emergence Herbicidal Evaluation of Test Compounds | | | | | | | | |
|---|---|---|---|---|---|---|---|---|
| Example | Rate | A | A | I | E | P | G | Z |
| Number | kg/ha | B | M | P | C | A | O | E |
| | | U | B | O | H | N | S | A |
| | | T | E | H | C | D | H | M |
| | | H | L | E | G | I | I | X |
| 8 | 0.2500 | 9.0 | 9.0 | | 9.0 | 9.0 | 9.0 | 5.0 |
| | 0.1250 | 9.0 | 9.0 | | 9.0 | 9.0 | 9.0 | 0.0 |
| | | | | | | | | |
| 17 | 0.2000 | 4.0 | 1.0 | 3.0 | 2.0 | 2.0 | | 0.0 |
| | | | | | | | | |
| 18 | 0.2000 | 1.0 | 3.0 | 0.0 | 1.0 | 3.0 | | 0.0 |
| | | | | | | | | |
| 19 | 0.2000 | 2.0 | 0.0 | 1.0 | 4.0 | 4.0 | | 0.0 |
| | | | | | | | | |
| 20 | 0.2000 | 0.0 | 1.0 | 1.0 | 2.0 | 2.0 | | 0.0 |
| | | | | | | | | |
| 21 | 0.2000 | 7.0 | 7.0 | 7.0 | 9.0 | 9.0 | | 2.0 |
| | 0.1000 | 4.0 | 4.0 | 6.0 | 5.0 | 6.0 | | 1.0 |

**Table 6**

| Pre-emergence Herbicidal Evaluation of Test Compounds | | | | | | | | | |
|---|---|---|---|---|---|---|---|---|---|
| Example | Rate | A | A | I | M | D | E | S | Z |
| Number | kg/ha | B | M | P | A | I | C | E | E |
| | | U | B | O | T | G | H | T | A |
| | | T | E | H | I | S | C | V | M |
| | | H | L | E | N | A | G | I | X |
| 9 | 0.4000 | 8.0 | | 7.0 | 9.0 | | 4.0 | 9.0 | 1.5 |
| | 0.1000 | 5.0 | | 3.0 | 9.0 | | 3.0 | 5.0 | 1.5 |
| | 0.0250 | 4.0 | | 3.0 | 8.0 | | 1.0 | 2.0 | 1.0 |
| | | | | | | | | | |
| 10 | 0.4000 | 9.0 | | 9.0 | 9.0 | 9.0 | 9.0 | 9.0 | 5.0 |
| | 0.1000 | 9.0 | | 9.0 | 9.0 | 8.0 | 7.0 | 9.0 | 4.0 |
| | 0.0250 | 9.0 | | 9.0 | 8.0 | | 6.0 | 8.0 | 3.0 |
| | | | | | | | | | |
| 11 | 0.4000 | 4.0 | 6.0 | 2.0 | 8.0 | | 4.0 | 6.0 | 0.5 |
| | 0.1000 | 2.0 | 6.0 | 1.0 | 8.0 | | 2.0 | 3.0 | 0.5 |
| | 0.0250 | 1.0 | 3.0 | 1.0 | 4.0 | | 1.0 | 2.0 | 0.0 |
| | | | | | | | | | |
| 12 | 0.4000 | 9.0 | | 9.0 | 8.0 | | 9.0 | 9.0 | 4.0 |
| | 0.1000 | 4.0 | | 6.0 | 8.0 | | 7.0 | 9.0 | 3.0 |
| | 0.0250 | 4.0 | | 6.0 | 8.0 | | 6.0 | 9.0 | 2.0 |
| | | | | | | | | | |
| 13 | 0.4000 | 5.0 | | 7.0 | 8.0 | | 7.0 | 9.0 | 2.5 |
| | 0.1000 | 3.0 | | 3.0 | 8.0 | | 5.0 | 9.0 | 1.0 |
| | 0.0250 | 2.0 | | 2.0 | 5.0 | | 2.0 | 6.0 | 1.0 |
| | | | | | | | | | |
| 14 | 0.4000 | 0.0 | 4.0 | 0.0 | 3.0 | | 0.0 | 0.0 | 0.0 |
| | 0.1000 | 0.0 | | 0.0 | 1.0 | | 0.0 | 0.0 | 0.0 |
| 15 | 0.4000 | 4.0 | | 8.0 | 8.0 | | 8.0 | 9.0 | 4.0 |
| | 0.1000 | 3.0 | | 7.0 | 8.0 | | 7.0 | 9.0 | 3.0 |
| | 0.0250 | 2.0 | | 3.0 | 8.0 | | 5.0 | 9.0 | 2.0 |
| | | | | | | | | | |
| 23 | 0.4000 | 9.0 | | 9.0 | 9.0 | | 8.0 | 9.0 | 4.0 |
| | 0.1000 | 7.0 | | 5.0 | 8.0 | | 7.0 | 9.0 | 3.0 |
| | 0.0250 | 6.0 | | 4.0 | 8.0 | | 6.0 | 9.0 | 2.0 |
| | | | | | | | | | |
| 24 | 0.4000 | 9.0 | 9.0 | 9.0 | 8.0 | | 9.0 | 9.0 | 4.0 |
| | 0.1000 | 6.0 | 8.0 | 3.0 | 8.0 | | 8.0 | 9.0 | 2.5 |
| | 0.0250 | 5.0 | 7.0 | 3.0 | 8.0 | | 6.0 | 9.0 | 2.0 |

The post-emergence herbicidal activity of the compounds of the present invention is demonstrated by the following test, wherein a variety of monocotyledonous and dicotyledonous plants are treated with formulations prepared from solutions of the test compounds in acetone containing 0,4 % by weight of an alkylphenol/ethylene oxide condensate available under the trade mark TRITON X-155. These acetone solutions were diluted with water and the resulting formulations applied at dosage levels equivalent of about 0.4 to 0.2 kg per hectare of test compound per pot. After spraying the plants are placed on greenhouse benches and are cared for in the usual manner, commensurate with conventional greenhouse practices. From 2 to 4 weeks after treatment, the seedling plants are examined and rated according to the rating system provided below.

| | % Control |
|---|---|
| Rating | (As Compared To Check) |
| 9 | 100 |
| 8 | 91-99 |
| 7 | 80-90 |
| 6 | 65-79 |
| 5 | 45-64 |
| 4 | 30-44 |
| 3 | 16-29 |
| 2 | 6-15 |
| 1 | 1-5 |
| 0 | No Effect |

The results of the tests are set out in Tables 7 to 8 below. The compounds of the invention showed good performance on monocotyledonous and dicotyledonous weeds:

**Table 7**

| Post-emergence Herbicidal Evaluation of Test Compounds | | | | | | |
|---|---|---|---|---|---|---|
| Example | Rate | A | A | I | E | P |
| Number | kg/ha | B | M | P | C | A |
| | | U | B | O | H | N |
| | | T | E | H | C | D |
| | | H | L | E | G | I |
| 8 | 0.2500 | 9.0 | 9.0 | | 9.0 | 9.0 |
| 17 | 0.2000 | 4.0 | 4.0 | 2.0 | 3.0 | 2.0 |
| 18 | 0.2000 | 4.0 | 3.0 | 4.0 | 2.0 | 3.0 |
| 19 | 0.2000 | 3.0 | 3.0 | 4.0 | 3.0 | 2.0 |
| 20 | 0.2000 | 2.0 | 3.0 | 4.0 | 3.0 | 3.0 |
| 21 | 0.2000 | 7.0 | 7.0 | 7.0 | 6.0 | 5.0 |

**Table 8**

| Post-emergence Herbicidal Evaluation of Test Compounds | | | | | | | | | | |
|---|---|---|---|---|---|---|---|---|---|---|
| Example | Rate | A | A | G | I | L | M | D | E | S |
| Number | kg/ha | B | M | A | P | A | A | I | C | E |
| | | U | B | L | O | M | T | G | H | T |
| | | T | E | A | H | P | I | S | C | V |
| | | H | L | P | E | U | N | A | G | I |
| 9 | 0.400 | 5.0 | | 4.0 | 8.0 | | 6.0 | | 4.0 | 6.0 |
| 10 | 0.400 | 8.0 | 6.0 | 7.5 | 9.0 | 7.5 | 6.5 | 7.5 | 8.5 | 7.5 |
| 11 | 0.400 | 5.0 | 4.0 | 6.0 | 9.0 | 8.0 | 8.0 | 3.0 | 5.0 | 7.0 |
| 12 | 0.400 | 7.0 | 6.0 | 7.0 | 9.0 | 7.0 | 7.0 | 8.0 | 8.0 | 6.0 |
| 13 | 0.400 | 6.0 | 5.0 | 7.0 | 9.0 | 7.0 | 7.0 | 7.0 | 7.0 | 7.0 |
| 14 | 0.400 | 0.0 | 4.0 | 2.0 | 3.0 | 3.0 | 3.0 | 0.0 | 0.0 | 0.0 |
| 15 | 0.400 | 5.0 | 7.0 | 8.0 | 9.0 | 8.0 | 8.0 | | | 8.0 |
| 23 | 0.400 | 7.0 | 6.0 | 7.0 | 9.0 | 7.0 | 7.0 | 7.0 | 8.0 | 6.0 |
| 24 | 0.400 | 7.0 | 6.0 | 8.0 | 9.0 | 7.0 | 7.0 | 7.0 | 8.0 | 7.0 |

## Claims

1. A compound of formula I 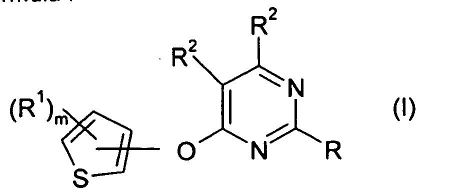 wherein
R¹ each independently represent a halogen atom; an alkyl, alkoxy, alkoxyalkyl, alkoxyalkoxy, alkoxycarbonyl or alkanoyloxy group, which may be substituted by phenyl, halogen, nitro, cyano, hydroxyl, C₁-C₄-alkoxy, C₁-C₄-haloalkoxy or C₁-C₄-alkoxycarbonyl; an alkenyl, alkynyl, haloalkyl, haloalkoxy, formyl, cyano or SF₅-group; a phenyl or naphthyl group, which may be substituted by halogen atoms, nitro, cyano, amino, hydroxy, phenoxy, C₁-C₄-alkyl, C₁-C₄-alkoxy, C₁-C₄-haloalkyl, C₁-C₄-haloalkenyl,
C₁-C₄-haloalkoxy, C₁-C₄-haloalkylthio, C₁-C₄-alkylsulfonyl or halosulfanyl; a -S(O)ₚ-R³group, in which p is 0, 1 or 2, and R³ represents an alkyl or haloalkyl group; -NR⁴R⁵, in which R⁴ and R⁵ each independently represent a hydrogen atom, an alkyl, alkenyl, aralkyl or aryl group;
R² each independently represent a hydrogen atom, a nitro group or has the meaning given for R¹;
R represents a group of formula Z-B, wherein;
Z represents a single bond, an oxygen or sulfur atom or a -OCH₂- or -SCH₂-group;
B represents an phenyl, naphthyl or a 5- or 6-membered nitrogen-containing heteroaryl or a thienyl group, which may be substituted by halogen atoms, nitro, cyano, amino, hydroxy, phenoxy, C₁-C₄-alkyl, C₁-C₄-alkoxy, C₁-C₄-haloalkyl, C₁-C₄-haloalkenyl, C₁-C₄-haloalkoxy, C₁-C₄-haloalkylthio, C₁-C₄-alkylsulfonyl or halosulfanyl; and m is 0 or an integer from 1 to 3;
wherein if any of above mentioned moietys comprises an alky, alkenyl or alkynyl group, such groups may be linear or branched and contain 1 to 6 carbon atoms;
wherein the alkyl portion of a haloalkyl, haloalkoxy, or alkoxy group has from 1 to 4 carbon atoms; and
wherein the number of carbon atoms in the alkoxyalkyl, alkoxyalkoxy or dialkoxyalkyl group is up to 6.

2. A compound according to claim 1 wherein R¹ each independently represent a halogen atom or a haloalkyl group.

3. A compound of formula IA 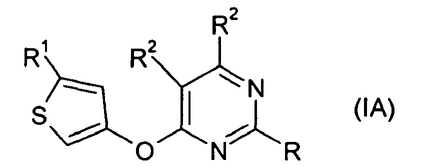 wherein R¹, R and R² have the meaning given in claim 1 or 2.

4. A compound as claimed in claims 1 or 3, wherein Z represents an oxygen atom or a -OCH₂- group.

5. A compound according to claim 3 selected from the group consisting of
2,4-bis(5-trifluoromethylthienyl-3-oxy)-6-methoxypyrimidine,
2-benzyloxy-6-methoxy-4-(5-trifluoromethylthienyl-3-oxy)-pyrimidine,

6. A compound having the structure of formula 12 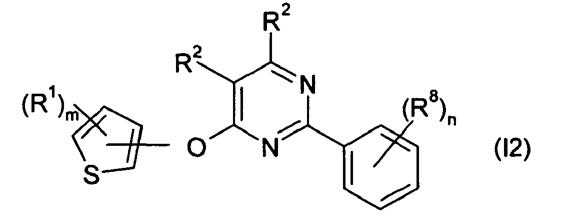 R¹, R² and m are as defined in any of claims 1 to 3;
n is 0 or an integer from 1 to 5; and
R⁸ each independently represent a halogen atom, a C₁-C₆alkyl group optionally substituted with one or more halogen atom, C₁-C₄alkoxy, C₁-C₄haloalkoxy, C₁-C₄alkoxycarbonyl, OH, CX'R⁹, CN, NO₂, R¹⁰S(O)_{y} or phenyl groups,
a C₂-C₆alkenyl group optionally substituted with one or more halogen atom, C₁-C₄alkoxy, C₁-C₄haloalkoxy, C₁-C₄alkoxycarbonyl, OH, CX'R⁹, CN, NO₂, R¹⁰S(O)_{y} or phenyl groups, a C₃-C₆alkynyl group optionally substituted with one or more halogen atom, C₁-C₄alkoxy, C₁-C₄haloalkoxy, C₁-C₄alkoxycarbonyl, OH, CX'R⁹, CN, NO₂, R¹⁰S(O)_{y} or phenyl groups, a C₁-C₆alkoxy group optionally substituted with one or more halogen atom, C₁-C₄alkoxy, C₁-C₄haloalkoxy, C₁-C₄alkoxycarbonyl, OH, CX'R⁹, CN, NO₂, R¹⁰S(O)_{y} or phenyl groups, or R¹⁰S(O)_{y}, NR¹¹R¹², NO₂, CN, CX'R⁹, CO₂R¹³ or OCOR¹⁴;
X¹ is each independently O, NOR¹⁵ or NNR¹⁶R¹⁷;
R⁹, R¹³, R¹⁴ and R¹⁶ are each independently H or C₁-C₄alkyl optionally substituted with one or more halogen, phenyl or furyl groups;
R¹⁰, R¹¹, R¹², R¹⁶ and R¹⁷ are each independently H, C₁-C₄alkyl or C₁-C₄alkoxy, and
y is each independently 0 or an integer of 1 or 2.

7. A compound according to claim 6 having the structure of formula IA2 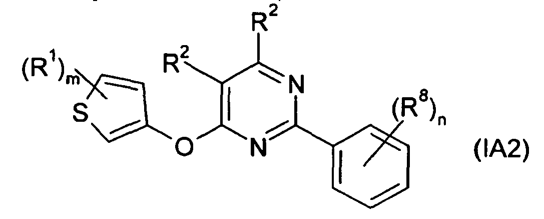 wherein R¹, R², R⁸, m and n are as defined in claim 6.

8. A compound according to claim 7 wherein m is 1; R¹ is attached to the 5-position of the thienyloxy ring; n is 1; and R⁸ is attached to the 4-position of the phenyl group.

9. A compound according to claim 3 selected from the group consisting of
4-methyl-2-(α,α,α-trifluoro-p-tolyl)-6-{[5-(trifluoromethyl)-3-thienyl]oxy}-pyrimidine;
4-methoxy-2-(α,α,α-trifluoro-p-tolyl)-6-{[5-(trifluoromethyl-3-thienyl]oxy} pyrimidine;
5-ethyl-2-(α,α,α-trifluoro-p-tolyl)-6-{[5-(trifluoromethyl-3-thienyl]oxy}pyrimidine;
4-methyl-6-(α,α,α-trifluoro-p-tolyl)-2-{[5-(trifluoromethyl)-3-thienyl]oxy}pyrimidine;
5-methyl-2-(α,α,α-trifluoro-p-tolyl)-6-{[5-(trifluoromethyl-3-thienyl]oxy)pyrimidine;
5-ethyl-2-(α,α,α-trifluoro-p-tolyl)-6-{[5-(trifluoromethyl-sulfonyl)3-thienyl]oxy) pyrimidine;
4-ethyl-2-(α,α,α-trifluoro-p-tolyl)-6-{[5-(trifluoromethyl-3-thienyl]oxy}pyrimidine;
2-(α,α,α-trifluoro-p-tolyl)-6-{[5-(trifluoromethyl-3-thienyl]oxy}pyrimidine;
4-n-propyl-2-(α,α,α-trifluoro-p-tolyl)-6-{[5-(trifluoromethyl-3-thienyl]oxy}pyrimidine;
4-chloro-5-ethyl-2-(α,α,α-trifluoro-p-tolyl)-6-{[5-(trifluoromethyl-3-thienyl]oxy}pyrimidine;
4-chloro-2-(α,α,α-trifluoro-p-tolyl)-6-{[5-(trifluoromethyl-3-thienyl]oxy}pyrimidine;
4-(methoxymethyl)-2-(α,α,α-trifluoro-p-tolyl)-6-{[5-(trifluoromethyl-3-thienyl]oxy} pyrimidine;
4-methyl-2-(α,α,α-trifluoro-p-tolyl)-6-{[5-(hydroxymethyl)-3-thienyl]oxy}pyrimidine;
4-(difluoromethyl)-2-(α,α,α-trifluoro-p-tolyl)-6-{[5-(trifluoromethyl-3-thienyl]oxy} pyrimidine;
5-ethyl-2-(α,α,α-trifluoro-p-tolyl)-6-{[5-(trifluoromethylsulfonyl)-3-thienyl]oxy}pyrimidine;
5-methyl-2-(α,α,α-trifluoro-p-tolyl)-6-{[5-(methylsulfonyl)-3-thienyl]oxy}pyrimidine;
4-methoxy-2-(α,α,α-trifluoro-p-tolyl)-6-{[5-formyl-3-thienyl]oxy}pyrimidine;
4-ethyl-2-(α,α,α-trifluoro-p-tolyl)-6-{[5-(methylsulfonyl)-3-thienyl]oxy}pyrimidine;
4-methyl-2-(α,α,α-trifluoro-p-tolyl)-6-{[5-(trifluoromethylsulfonyl)-3-thienyl]oxy}pyrimidine;
4-cyano-2-(α,α,α-trifluoro-p-tolyl)-6-{[5-(methylsulfonyl)-3-thienyl]oxy}pyrimidine; and
5-methoxymethyl-2-(α,α,α-trifluoro-p-tolyl)-6-{[5-(trifluoromethylsulfonyl)-3-thienyl]oxy}pyrimidine;
4-fluoro-2-(α,α,α-trifluoro-p-tolyl)-6-([5-(trifluoromethyl)-3-thienyl]oxy}pyrimidine;
4-methylamino-2-(α,α,α-trifluoro-p-tolyl)-6-{[5-(trifluoromethyl)-3-thienyl]oxy}pyrimidine;
4-methyl-2-(4-chlorophenyl)-6-{[5-(trifluoromethyl)-3-thienyl]oxy}pyrimidine,
5-methyl-4-fluoro-2-(α,α,α-trifluoro-p-tolyl)-6-{[5-(trifluoromethyl)-3-thienyl]oxy}pyrimidine;
5-methyl-2-(4-chlorophenyl)-6-{[5-(trifluoromethyl)-3-thienyl]oxy}pyrimidine,
2-(4-chlorophenyl)-6-{[5-(trifluoromethyl)-3-thienyl]oxy}pyrimidine,
4-methyl-2-(4-tert-butylphenyl)-6-{[5-(trifluoromethyl)-3-thienyl]oxy}pyrimidine,
4-methyl-2-(3-thienyl)-6-{[5-(trifluoromethyl)-3-thienyl]oxy}pyrimidine,
5-methyl-2-(2-thienyl)-6-{[5-(trifluoromethyl)-3-thienyl]oxy}pyrimidine,
2-(5-chloro-2-thienyl)-6-{[5-(trifluoromethyl)-3-thienyl]oxy}pyrimidine,
4-methoxy-2-(5-chloro-3-thienyl)-6-{[5-(trifluoromethyl)-3-thienyl]oxy}pyrimidine,
4-methyl-2-(5-trifluoromethyl-2-thienyl)-6-{[5-(trifluoromethyl)-3-thienyl]oxy}pyrimidine and
2-(5-trifluoromethyl-3-thienyl)-6-{[5-(trifluoromethyl)-3-thienyl]oxy}pyrimidine.

10. A process for the preparation of a compound of formula I or I2 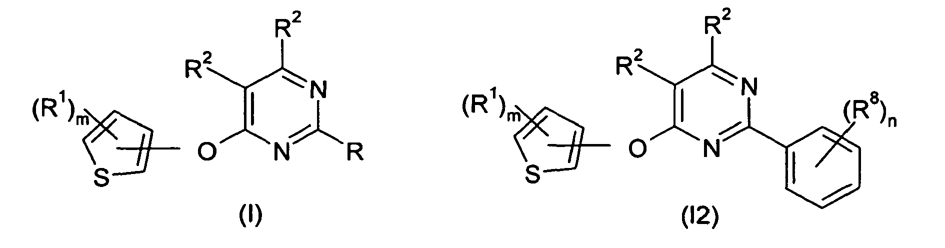 wherein R, R¹, R² and m are as defined in claims 1 to 10, and R⁸ and n are defined as in claim 6, which comprises reacting a compound of formula II 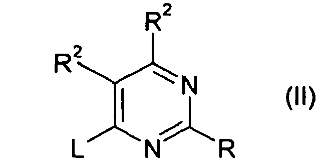 in which Rand R² are as defined hereinabove for formula I or 12, and L represents a leaving group, with a compound of formula III or a metal salt thereof 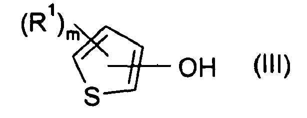 in which R¹ and m are as defined hereinabove for formula I.

11. A compound of formula IIIA 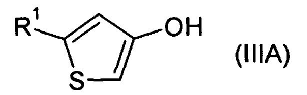 wherein R¹ is as defined hereinabove for formula I.

12. A compound according to claim 11 wherein R¹ represents a trifluoromethyl group.

13. A process for the preparation of a compound of formula IIIA 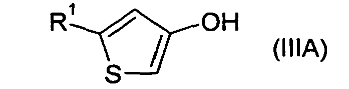 in which R¹ is as defined hereinabove for formula I, which comprises the steps of
(a) saponification of a compound of formula XIIIA 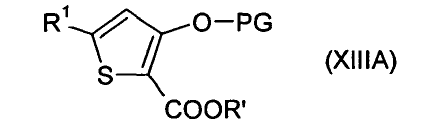 in which R¹ is as described for formula IIIA; R' is C₁₋₆ alkyl; and PG represents a hydrogen atom or a protecting group, to obtain a compound of formula XIVA 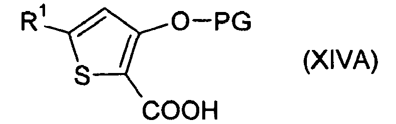
(b) decarboxylation of the compound of formula XIVA; and
(c) optionally cleaving the protecting group.

14. A herbicidal composition which comprises an inert solid or liquid carrier and a herbicidally effective amount having the structure of formula I or I2 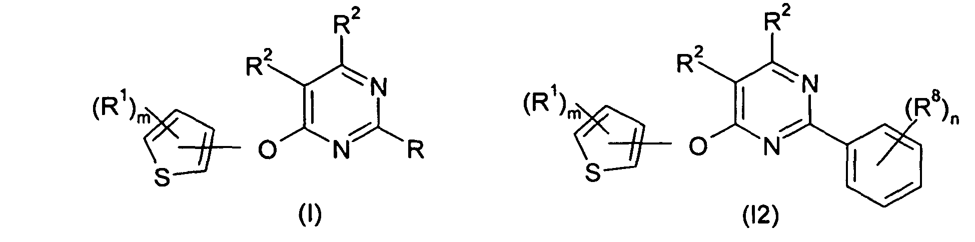 wherein R, R¹, R² and m are as defined in claim 1 or 2, and R⁸ and n are as defined in claim 6.

15. A method for the control of monocotyledenous and dicotyledenous annual, perennial and aquatic plant species which comprises applying to the foliage of said plants, or to the soil or water containing the seeds or other propagating organs thereof a herbicidally effective amount of the compound of formula I or I2 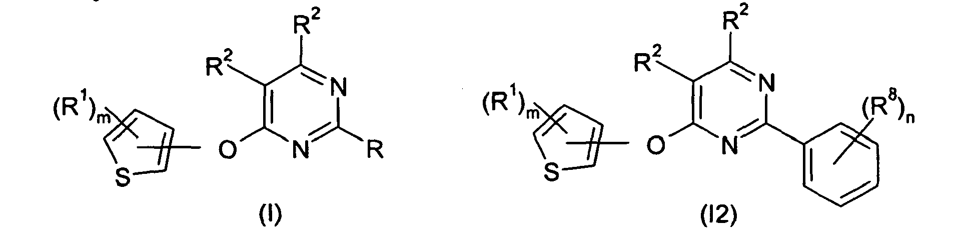 wherein R, R¹, R² and m are as defined in claims 1 to 4, and R⁸ and n are as defined in claim 6.

## Patentansprüche

1. Verbindungen der Formel I 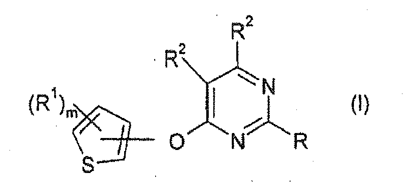 wobei
R¹ jeweils unabhängig für ein Halogenatom; eine Alkyl-, Alkoxy-, Alkoxyalkyl-, Alkoxyalkoxy-, Alkoxycarbonyl- oder Alkanoyloxygruppe, die durch Phenyl, Halogen, Nitro, Cyano, Hydroxy, C₁-C₄-Alkoxy, C₁-C₄-Halogenalkoxy oder C₁-C₄-Alkoxycarbonyl substituiert sein kann; eine Alkenyl-, Alkinyl-, Halogenalkyl-, Halogenalkoxy-, Formyl-, Cyano- oder SF₅-Gruppe; eine Phenyl- oder Naphthylgruppe, die durch Halogenatome, Nitro, Cyano, Amino, Hydroxy, Phenoxy, C₁-C₄-Alkyl, C₂-C₄-Alkoxy, C₁-C₄-Halogenalkyl, C₁-C₄-Halogenalkenyl, C₁-C₄-Halogenalkoxy, C₁-C₄-Halogenalkylthio, C₁-C₄-Alkylsulfonyl oder Halogensulfanyl substituiert sein kann; eine -S(O)ₚ-R³-Gruppe, in welcher p für 0, 1 oder 2 steht und R³ für eine Alkyl- oder Halogenalkylgruppe steht; -NR⁴R⁵, wobei R⁴ und R⁵ jeweils unabhängig für ein Wasserstoffatom, eine Alkyl-, Alkenyl-, Aralkyl- oder Arylgruppe stehen, steht;
R² jeweils unabhängig für ein Wasserstoffatom oder eine Nitrogruppe steht oder die für R¹ angegebene Bedeutung aufweist;
R für eine Gruppe der Formel Z-B steht, wobei
Z für eine Einfachbindung, ein Sauerstoff- oder Schwefelatom oder eine -OCH₂- oder -SCH₂-Gruppe steht;
B für eine Phenyl-, Naphthyl- oder eine 5- oder 6gliedrige stickstoffhaltige Heteroaryl- oder eine Thienylgruppe steht, die durch Halogenatome, Nitro, Cyano, Amino, Hydroxy, Phenoxy, C₁-C₄-Alkyl, C₁-C₄-Alkoxy, C₁-C₄-Halogenalkyl, C₁-C₄-Halogenalkenyl, C₁-C₄-Halogenalkoxy, C₁-C₄-Halogenalkylthio, C₁-C₄-Alkylsulfonyl oder Halogensulfanyl substituiert sein kann; und m für 0 oder eine ganze Zahl von 1 bis 3 steht;
wobei, wenn eine der obenerwähnten Molekülgruppen eine Alkyl-, Alkenyl- oder Alkinylgruppe enthält, diese Gruppen geradkettig oder verzweigt sein können und 1 bis 6 Kohlenstoffatome enthalten;
wobei der Alkylteil einer Halogenalkyl-, Halogenalkoxy- oder Alkoxygruppe 1 bis 4 Kohlenstoffatome aufweist; und
wobei die Anzahl von Kohlenstoffatomen in der Alkoxyalkyl-, Alkoxyalkoxy- oder Dialkoxyalkylgruppe bis zu 6 beträgt.

2. Verbindungen nach Anspruch 1, wobei R¹ jeweils unabhängig für ein Halogenatom oder eine Halogenalkylgruppe steht.

3. Verbindungen der Formel IA 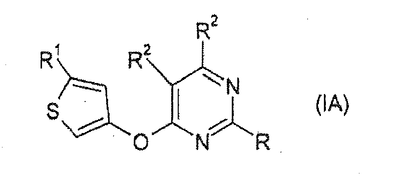 wobei R¹, R und R² die in Anspruch 1 oder 2 angegebene Bedeutung aufweisen.

4. Verbindungen nach Anspruch 1 oder 3, wobei Z für ein Sauerstoffatom oder eine -OCH₂-Gruppe steht.

5. Verbindungen nach Anspruch 3, ausgewählt aus der aus 2,4-Bis(5-trifluormethylthzenyl-3-oxy)-6-methoxypyrimidin und 2-Benzyloxy-6-methoxy-4-(5-trifluormethylthienyl-3-oxy)pyrimidin bestehenden Gruppe.

6. Verbindungen mit der Struktur der Formel I2 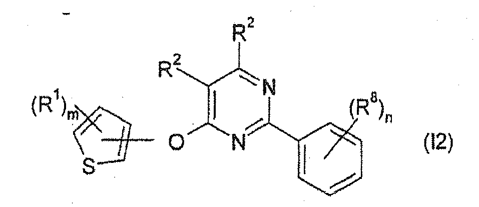 wobei
R¹, R² und m wie in einem der Ansprüche 1 bis 3 definiert sind;
n für 0 oder eine ganze Zahl von 1 bis 5 steht; und
R⁸ jeweils unabhängig für ein Halogenatom, eine C₁-C₆-Alkylgruppe, gegebenenfalls substituiert durch ein oder mehrere Halogenatome, C₁-C₄-Alkoxy, C₁-C₄-Halogenalkoxy-, C₁-C₄-Alkoxycarbonyl-, OH-, CX'R⁹-, CN-, NO₂-, R¹⁰S(O)_{y}- oder Phenylgruppen,
eine C₂-C₆-Alkenylgruppe, gegebenenfalls substituiert durch ein oder mehrere Halogenatome, C₁-C₄-Alkoxy-, C₂-C₄-Halogenalkoxy-, C₁-C₄-Alkoxycarbonyl-, OH-, CX'R⁹-, CN-, NO₂-, R¹⁰S(O)_{y}- oder Phenylgruppen, eine C₃-C₆-Alkinylgruppe, gegebenenfalls substituiert durch ein oder mehrere Halogenatome, C₁-C₄-Alkoxy-, C₁-C₄-Halogenalkoxy-, C₁-C₄-Alkoxycarbonyl-, OH-, CX'R⁹-, CN-, NO₂-, R¹⁰S(O)_{y}- oder Phenylgruppen, eine C₁-C₆-Alkoxygruppe, gegebenenfalls substituiert durch ein oder mehrere Halogenatome, C₁-C₄-Alkoxy-, C₁-C₄-Halogenalkoxy-, C₁-C₄-Alkoxycarbonyl-, OH-, CX'R⁹-, CN-, NO₂-, R¹⁰S(O)_{y}- oder Phenylgruppen, oder R¹⁰S(O)_{y}, NR¹¹R¹², NO₂, CN, CX'R⁹, CO₂R¹³ oder OCOR¹⁴ steht;
X¹ jeweils unabhängig für O, NOR¹⁵ oder NNR¹⁶R¹⁷ steht;
R⁹, R¹³, R¹⁴ und R¹⁶ jeweils unabhängig für H oder C₁-C₄-Alkyl, gegebenenfalls substituiert durch eine oder mehrere Halogen-, Phenyl- oder Furylgruppen, steht;
R¹⁰, R¹¹, R¹², R¹⁶ und R¹⁷ jeweils unabhängig voneinander für H, C₁-C₄-Alkyl oder C₁-C₄-Alkoxy stehen,
und
y jeweils unabhängig für 0 oder eine ganze Zahl 1 oder 2 steht.

7. Verbindungen nach Anspruch 6 mit der Struktur der Formel IA2 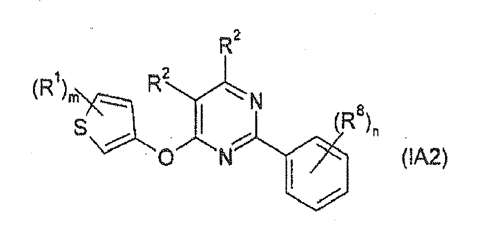 wobei R¹, R², R⁸, m und n wie in Anspruch 6 definiert sind.

8. Verbindungen nach Anspruch 7, wobei m für 1 steht; R¹ in der 5-Stellung des Thienyloxyrings gebunden ist; n für 1 steht und R⁸ in der 4-Stellung der Phenylgruppe gebunden ist.

9. Verbindungen nach Anspruch 3, ausgewählt aus der aus
4-Methyl-2-(α,α,α-trifluor-p-tolyl)-6-{[5-(trifluormethyl)-3-thienyl]oxy}pyrimidin;
4-Methoxy-2-(α,α,α-trifluor-p-tolyl)-6-{[5-(trifluormethyl-3-thienyl]oxy}pyrimidin;
5-Ethyl-2-(α,α,α-trifluor-p-tolyl)-6-{[5-(trifluormethyl-3-thienyl]oxy}pyrimidin;
4-Methyl-6-(α,α,α-trifluor-p-tolyl)-2-{[5-(trifluormethyl)-3-thienyl]oxy}pyrimidin;
5-Methyl-2-(α,α,α-trifluor-p-tolyl)-6-{[5-(trifluormethyl-3-thienyl]oxy)pyrimidin;
5-Ethyl-2-(α,α,α-trifluor-p-tolyl)-6-{[5-(trifluormethylsulfonyl)-3-thienyl]oxy}pyrimidin;
4-Ethyl-2-(α,α,α-trifluor-p-tolyl)-6-{[5-(trifluormethyl-3-thienyl]oxy}pyrimidin;
2-(α,α,α-Trifluor-p-tolyl)-6-{[5-(trifluormethyl-3-thienyl]oxy}pyrimidin;
4-n-Propyl-2-(α,α,α-trifluor-p-tolyl)-6-{[5-(trifluormethyl-3-thienyl]oxy}pyrimidin;
4-Chlor-5-ethyl-2-(α,α,α-trifluor-p-tolyl)-6-{[5-(trifluormethyl-3-thienyl]oxy}pyrimidin;
4-Chlor-2-(α,α,α-trifluor-p-tolyl)-6-{[5-(trifluormethyl-3-thienyl]oxy}pyrimidin;
4-(Methoxymethyl)-2-(α,α,α-trifluor-p-tolyl)-6-{[5-(trifluormethyl-3-thienyl]oxy}pyrimidin;
4-Methyl-2-(α,α,α-trifluor-p-tolyl)-6-{[5-(hydroxymethyl)-3-thienyl]oxy}pyrimidin;
4-(Difluormethyl)-2-(α,α,α-trifluor-p-tolyl)-6-{[5-(trifluormethyl-3-thienyl]oxy}pyrimidin;
5-Ethyl-2-(α,α,α-trifluor-p-tolyl)-6-{[5-(trifluormethylsulfonyl)-3-thienyl]oxy}pyrimidin;
5-Methyl-2-(α,α,α-trifluor-p-tolyl)-6-{[5-(methylsulfonyl)-3-thienyl]oxy}pyrimidin;
4-Methoxy-2-(α,α,α-trifluor-p-tolyl)-6-{[5-formyl-3-thienyl]oxy}pyrimidin;
4-Ethyl-2-(α,α,α-trifluor-p-tolyl)-6-{[5-(methylsulfonyl)-3-thienyl]oxy}pyrimidin;
4-Methyl-2-(α,α,α-trifluor-p-tolyl)-6-{[5-(trifluormethylsulfonyl)-3-thienyl]oxy}pyrimidin;
4-Cyano-2-(α,α,α-trifluor-p-tolyl)-6-{[5-(methylsulfonyl)-3-thienyl]oxy}pyrimidin;
und
5-Methoxymethyl-2-(α,α,α-trifluor-p-tolyl)-6-{[5-(trifluormethylsulfonyl)-3-thienyl]oxy}pyrimidin;
4-Fluor-2-(α,α,α-trifluor-p-tolyl)-6-{[5-(trifluormethyl)-3-thienyl]oxy}pyrimidin;
4-Methylamino-2-(α,α,α-trifluor-p-tolyl)-6-{[5-(trifluormethyl)-3-thienyl]oxy}pyrimidin;
4-Methyl-2-(4-chlorphenyl)-6-{[5-(trifluormethyl)-3-thienyl]oxy}pyrimidin;
5-Methyl-4-fluor-2-(α,α,α-trifluor-p-tolyl)-6-{[5-(trifluormethyl)-3-thienyl]oxy}pyrimidin;
5-Methyl-2-(4-chlorphenyl)-6-{[5-(trifluormethyl)-3-thienyl]oxy}pyrimidin;
2-(4-Chlorphenyl)-6-{[5-(trifluormethyl)-3-thienyl]oxy}pyrimidin;
4-Methyl-2-(4-tert.-butylphenyl)-6-{[5-(trifluormethyl)-3-thienyl]oxy}pyrimidin;
4-Methyl-2-(3-thienyl)-6-{[5-(trifluormethyl)-3-thienyl]oxy}pyrimidin;
5-Methyl-2-(2-thienyl)-6-{[5-(trifluormethyl)-3-thienyl]oxy}pyrimidin;
2-(5-Chlor-2-thienyl)-6-{[5-(trifluormethyl)-3-thienyl]oxy}pyrimidin;
4-Methoxy-2-(5-chlor-3-thienyl)-6-{[5-(trifluormethyl)-3-thienyl]oxy}pyrimidin;
4-Methyl-2-(5-trifluormethyl-2-thienyl)-6-{[5-(trifluormethyl)-3-thienyl]oxy}pyrimidin; und
2-(5-Trifluormethyl-3-thienyl)-6-{[5-(trifluormethyl)-3-thienyl]oxy}pyrimidin bestehenden Gruppe.

10. Verfahren zur Herstellung von Verbindungen der Formel I oder 12 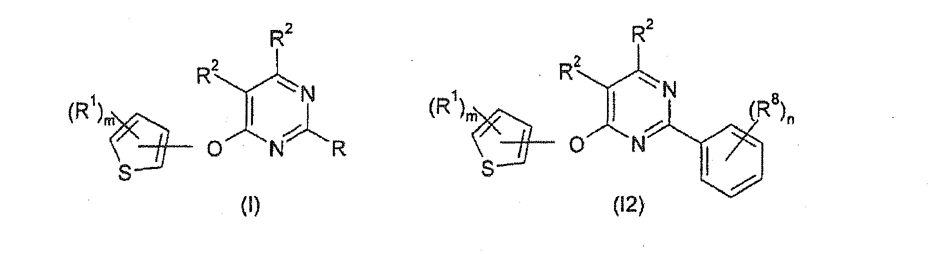 wobei R, R¹, R² und m wie in den Ansprüchen 1 bis 10 definiert sind und R⁸ und n wie in Anspruch 6 definiert sind, bei dem man eine Verbindung der Formel II 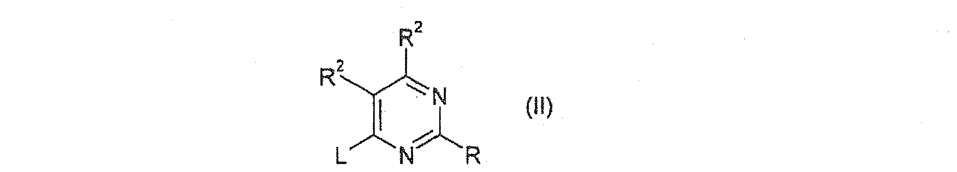 in welcher R und R² wie oben für die Formel I oder 12 definiert sind und L für eine Abgangsgruppe steht, mit einer Verbindung der Formel III oder einem Metallsalz davon 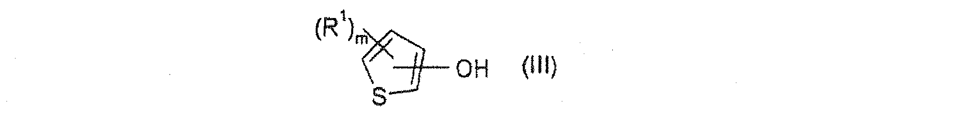 in welcher R¹ und m wie oben für Formel I definiert sind, umsetzt.

11. Verbindungen der Formel IIIA 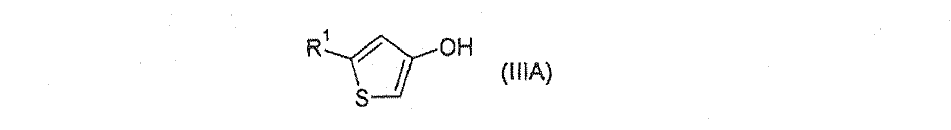 in welcher R¹ wie oben für Formel I definiert ist.

12. Verbindungen nach Anspruch 11, wobei R¹ für eine Trifluormethylgruppe steht.

13. Verfahren zur Herstellung einer Verbindung der Formel IIIA 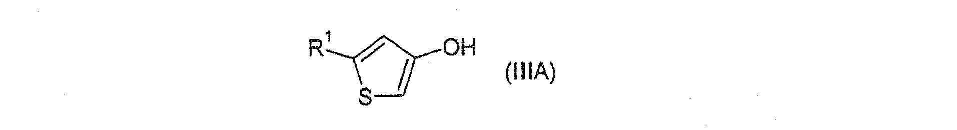 in welcher R¹ wie oben für Formel I definiert ist, bei dem man
(a) eine Verbindung der Formel XIIIA 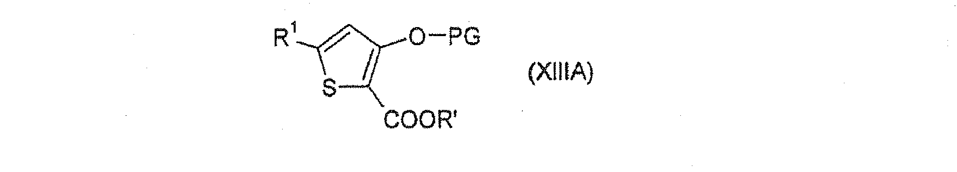 in welcher R¹ wie für Formel IIIA beschrieben ist; R' für C₁₋₆-Alkyl steht und PG für ein Wasserstoffatom oder eine Schutzgruppe steht, zu einer Verbindung der Formel XIVA 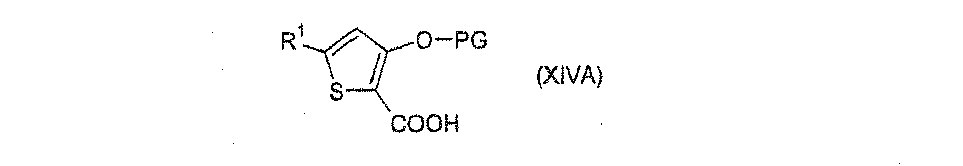 verseift;
(b) die Verbindung der Formel XIVA decarboxyliert; und
(c) gegebenenfalls die Schutzgruppe abspaltet.

14. Herbizide Zusammensetzung, enthaltend einen inerten festen oder flüssigen Träger und eine herbizid wirksame Menge der Struktur der Formel I oder 12 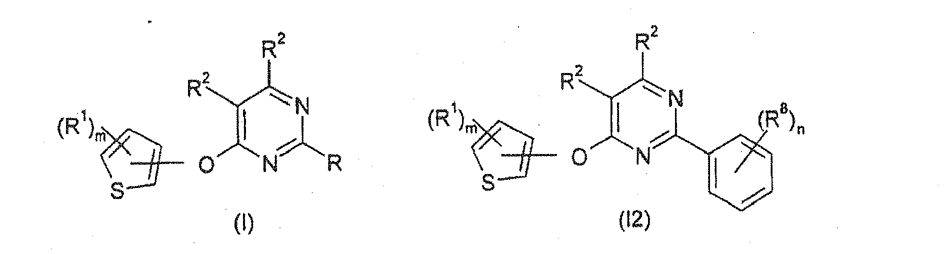 wobei R, R¹, R² und m wie in Anspruch 1 oder 2 definiert sind und R⁸ und n wie in Anspruch 6 definiert sind.

15. Verfahren zur Bekämpfung monocotyledoner und dicotyledoner einjähriger, mehrjähriger und aquatischer Pflanzenspezies, bei dem man auf die Blätter dieser Pflanzen oder den Boden oder Wasser, enthaltend die Samen oder andere Fortpflanzungsorgane davon, eine herbizid wirksame Menge der Verbindung der Formel I oder I2 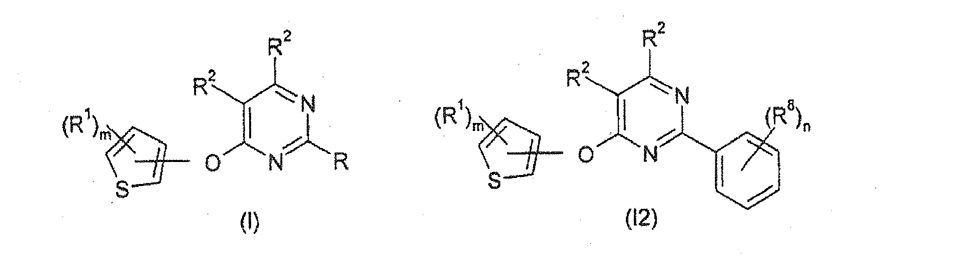 wobei R, R¹, R² und m wie in den Ansprüchen 1 bis 4 definiert sind und R⁸ und n wie in Anspruch 6 definiert sind, aufbringt.

## Revendications

1. Composé de formule I 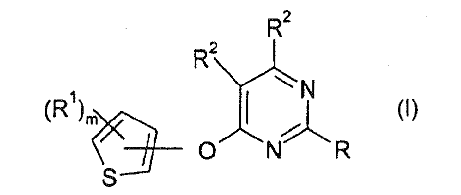 dans laquelle
R¹ représente de manière indépendante un atome d'halogène ; un groupe alkyle, alcoxy, alcoxyalkyle, alcoxyalcoxy, alcoxycarbonyle ou alcanoyloxy, qui peut être substitué par un groupe phényle, halogène, nitro, cyano, hydroxyle, alcoxy en C₁-C₄, halogénoalcoxy en C₁-C₄ ou alcoxycarbonyle en C₁-C₄ ; un groupe alcényle, alcynyle, halogénoalkyle, halogénoalcoxy, formyle, cyano or SF₅-; un groupe phényle or naphtyle, qui peut être substitué par des atomes d'halogène, un groupe nitro, cyano, amino, hydroxy, phénoxy, alkyle en C₁-C₄, alcoxy en C₁-C₄, halogénoalkyle en C₁-C₄, halogénoalcényle en C₁-C₄, halogénoalcoxy en C₁-C₄, halogénoalkylthio en C₁-C₄, alkylsulfonyle en C₁-C₄ ou halogénosulfonyle ; un groupe -S(O)ₚ-R³, dans lequel p vaut 0, 1 ou 2, et R³ représente un groupe alkyle ou halogénoalkyle ; un groupe -NR⁴R⁵, dans lequel R⁴ et R⁵ représentent indépendamment l'un de l'autre un atome d'hydrogène, un groupe alkyle, alcényle, aralkyle ou aryle ;
R² représente de manière indépendante un atome d'hydrogène, un groupe nitro ou a la signification donnée pour R¹ ;
R représente un groupe de formule Z-B, dans laquelle ;
Z représente une liaison simple, un atome d'oxygène ou de soufre ou un groupe -OCH₂- ou -SCH₂- ;
B représente un groupe phényle, naphtyle ou hétéroaryle pentagonal ou hexagonal contenant de l'azote ou thiényle, qui peut être substitué par des atomes d'halogène ou un groupe nitro, cyano, amino, hydroxy, phénoxy, alkyle en C₁-C₄, alcoxy en C₁-C₄, halogénoalkyle en C₁-C₄, halogénoalcényle en C₁-C₄, halogénoalcoxy en C₁-C₄, halogénoalkylthio en C₁-C₄, alkylsulfonyle en C₁-C₄ ou halogénosulfonyle ; et m vaut 0 ou un nombre entier de 1 à 3 ;
dans laquelle, si l'une quelconque des parties mentionnées ci-dessus comprend un groupe alkyle, alcényle ou alcynyle, ces groupes peuvent être linéaires ou ramifiés et contenir 1 à 6 atomes de carbone ; dans laquelle la portion alkyle d'un groupe halogénoalkyle, halogénoalcoxy ou alcoxy a de 1 à 4 atomes de carbone ; et
dans laquelle le nombre d'atomes de carbone dans le groupe alcoxyalkyle, alcoxyalcoxy ou dialcoxyalkyle peut aller jusqu'à 6.

2. Composé selon la revendication 1, dans lequel R¹ représente de manière indépendante un atome d'halogène ou un groupe halogénoalkyle.

3. Composé de formule IA 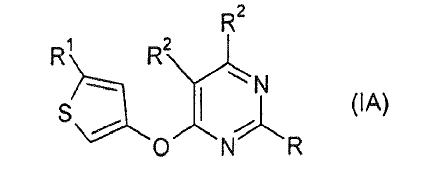 dans laquelle R¹, R et R² ont la signification donnée dans la revendication 1 ou 2.

4. Composé selon la revendication 1 ou 3, dans lequel Z représente un atome d'oxygène ou un groupe -OCH₂-.

5. Composé selon la revendication 3, sélectionné parmi le groupe constitué de la 2,4-bis(5-trifluorométhylthiényl-3-oxy)-6-méthoxypyrimidine et de la 2-benzyloxy-6-méthoxy-4-(5-trifluorométhylthiényl-3-oxy)-pyrimidine.

6. Composé ayant la structure de formule 12 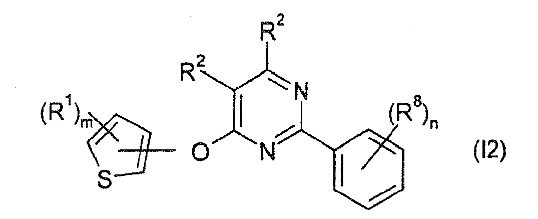 R¹, R² et m sont tels que définis dans l'une quelconque des revendications 1 à 3 :
n vaut 0 ou un nombre entier de 1 à 5 ; et
R⁸ représente de manière indépendante un atome d'halogène, un groupe alkyle en C₁-C₆ substitué de manière optionnelle par un ou plusieurs atomes d'halogène ou des groupes alcoxy en C₁-C₄, halogénoalcoxy en C₁-C₄, alcoxycarbonyle en C₁-C₄, OH, CX'R⁹, CN, NO₂, R¹⁰S(O)_{y} ou phényle, un groupe alcényle en C₂-C₆ substitué de manière optionnelle par un ou plusieurs atomes d'halogène ou des groupes alcoxy en C₁-C₄, halogénoalcoxy en C₁-C₄, alcoxycarbonyle en C₁-C₄, OH, CX'R⁹, CN, NO₂, R¹⁰S(O)_{y} ou phényle, un groupe alcényle en C₃-C₆ substitué de manière optionnelle par un ou plusieurs atomes d'halogène ou des groupes alcoxy en C₁-C₄, halogénoalcoxy en C₁-C₄, alcoxycarbonyle en C₁-C₄, OH, CX'R⁹, CN, NO₂, R¹⁰S(O)_{y} ou phényle, un groupe alcoxy en C₁-C₆ substitué de manière optionnelle par un ou plusieurs atomes d'halogène ou des groupes alcoxy en C₁-C₄, halogénoalcoxy en C₁-C₄, alcoxycarbonyle en C₁-C₄, OH, CX'R⁹, CN, NO₂, R¹⁰S(O)_{y} ou phényle, ou un groupe R¹⁰S(O)_{y}, NR¹¹R¹², NO², CN, CX'R⁹, CO₂R¹³ ou OCOR¹⁴ ;
X¹ est de manière indépendante O, NOR¹⁵ ou NNR¹⁶R¹⁷ ;
R⁹, R¹³, R¹⁴ et R¹⁶ sont indépendamment les uns des autres du H ou un groupe alkyle en C₁-C₄ substitué de manière optionnelle par un ou plusieurs groupes halogène, phényle ou furyle ;
R¹⁰, R¹¹, R¹², R¹⁶ et R¹⁷ sont indépendamment les uns des autres du H, un groupe alkyle en C₁-C₄ ou alcoxy en C₁-C₄,
et
Y vaut de manière indépendante 0 ou un nombre entier de 1 à 2.

7. Composé selon la revendication 6, ayant la structure de formule IA2 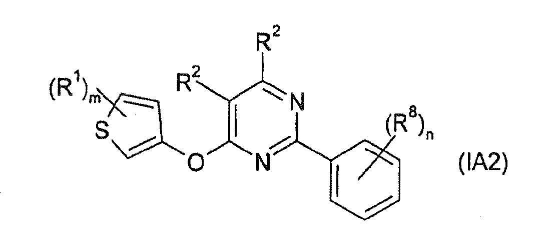 dans laquelle R¹, R², R⁸, m et n sont tels que définis dans la revendication 6.

8. Composé selon la revendication 7 dans lequel m vaut 1 ; R¹ est fixé à la position 5 du cycle thiényloxy ; n vaut 1 ; et R⁸ est fixé à la position 4 du groupe phényle.

9. Composé selon la revendication 3 sélectionné parmi le groupe constitué de la
4-méthyl-2-(α,α,α-trifluoro-p-tolyl)-6-{[5-(trifluorométhyl)-3-thiényl]oxy}-pyrimidine ;
4-méthoxy-2-(α,α,α-trifluoro-p-tolyl)-6-{[5-(trifluorométhyl-3-thiényl]oxy}pyrimidine ;
5-éthyl-2-(α,α,α-trifluoro-p-tolyl)-6-{[5-(trifluorométhyl-3-thiényl]oxy}pyrimidine ;
4-méthyl-6-(α,α,α-trifluoro-p-tolyl)-2-{[5-(trifluorométhyl)-3-thiényl]oxypyrimidine ;
5-méthyl-2-(α,α,α-trifluoro-p-tolyl)-6-{[5-(trifluorométhyl-3-thiényl]oxy)-pyrimidine ;
5-éthyl-2-(α,α,α-trifluoro-p-tolyl)-6-{[5-(trifluorométhyl-sulfonyl)3-thiényl]oxy)pyrimidine ;
4-éthyl-2-(α,α,α-trifluoro-p-tolyl)-6-{[5-(trifluorométhyl-3-thiényl]oxy}pyrimidine ;
2-(α,α,α-trifluoro-p-tolyl)-6-{[5-(trifluorométhyl-3-thiényl]-oxy}pyrimidine ;
4-n-propyl-2-(α,α,α-trifluoro-p-tolyl)-6-{[5-(trifluorométhyl-3-thiényl]oxy}pyrimidine ;
4-chloro-5-éthyl-2-(α,α,α-trifluoro-p-tolyl)-6-{[5-(trifluoro-méthyl-3-thiényl]oxy}pyrimidine ;
4-chloro-2-(α,α,α-trifluoro-p-tolyl)-6-{[5-(trifluorométhyl-3-thiényl]oxy}pyrimidine ;
4-(méthoxyméthyl)-2-(α,α,α-trifluoro-p-tolyl)-6-{[5-(trifluoro-méthyl-3-thiényl]oxy)pyrimidine ;
4-méthyl-2-(α,α,α-trifluoro-p-tolyl)-6-{[5-(hydroxyméthyl)-3-thiényl]oxy}pyrimidine ;
4-(difluorométhyl)-2-(α,α,α-trifluoro-p-tolyl)-6-{[5-(trifluorométhyl-3-thiényl]oxy}pyrimidine ;
5-éthyl-2-(α,α,α-trifluoro-p-tolyl)-6-{[5-(trifluorométhylsulfonyl)-3-thiényl]oxy}pyrimidine ;
5-méthyl-2-(α,α,α-trifluoro-p-tolyl)-6-{[5-(méthylsulfonyl)-3-thiényl]oxy}pyrimidine ;
4-méthoxy-2-(α,α,α-trifluoro-p-tolyl)-6-{[5-formyl-3-thiényl]-oxy}pyrimidine ;
4-éthyl-2-(α,α,α-trifluoro-p-tolyl)-6-{[5-(méthylsulfonyl}-3-thiényl]oxy}pyrimidine ;
4-méthyl-2-(α,α,α-trifluoro-p-tolyl)-6-{[5-(trifluorométhylsulfonyl)-3-thiényl]oxy}pyrimidine ;
4-cyano-2-(α,α,α-trifluoro-p-tolyl)-6-{[5-(méthylsulfonyl)-3-thiényl]oxy}pyrimidine ; et de la
5-méthoxyméthyl-2-(α,α,α-trifluoro-p-tolyl)-6-{[5-(trifluoro-méthylsulfonyl)-3-thiényl]oxy}pyrimidine ;
4-fluoro-2-(α,α,α-trifluoro-p-tolyl)-6-{[5-(trifluorométhyl)-3-thiényl]oxy}pyrimidine ;
4-méthylamino-2-(α,α,α-trifluoro-p-tolyl)-6-{[5-(trifluoro-méthyl)-3-thiényl]oxy}pyrimidine ;
4-méthyl-2-(4-chlorophényl)-6-{[5-(trifluorométhyl)-3-thiényl]-oxy}pyrimidine ;
5-méthyl-4-fluoro-2-(α,α,α-trifluoro-p-tolyl)-6-{(5-(trifluoro-méthyl)-3-thiényl]oxy}pyrimidine ;
5-méthyl-2-(4-chlorophényl)-6-{[5-(trifluorométhyl)-3-thiényl]-oxy}pyrimidine,
2-(4-chlorophényl)-6-{[5-(trifuorométhyl)-3-thiényl]oxy}-pyrimidine,
4-méthyl-2-(4-tert-butylphényl)-6-{[5-(trifluorométhyl)-3-thiényl]oxy}pyrimidine,
4-méthyl-2-(3-thiényl)-6-{(5-(trifluorométhyl)-3-thiényl]oxy}-pyrimidine,
5-méthyl-2-(2-thiényl)-6-{(5-(trifluorométhyl)-3-thiényl]oxy}-pyrimidine,
2-(5-chloro-2-thiényl)-6-{[5-(trifluorométhyl)-3-thiényl]oxy}-pyrimidine,
4-méthoxy-2-(5-chloro-3-thiényl)-6-{[5-(trifluorométhyl)-3-thiényl]oxy}pyrimidine,
4-méthyl-2-(5-trifluorométhyl-2-thiényl)-6-{[5-(trifluorométhyl)-3-thiényl]oxy}pyrimidine et de la
2-(5-trifluorométhyl-3-thiényl)-6-{[5-(trifluorométhyl)-3-thiényl]oxy}pyrimidine.

10. Procédé de préparation d'un composé de formule I ou I2 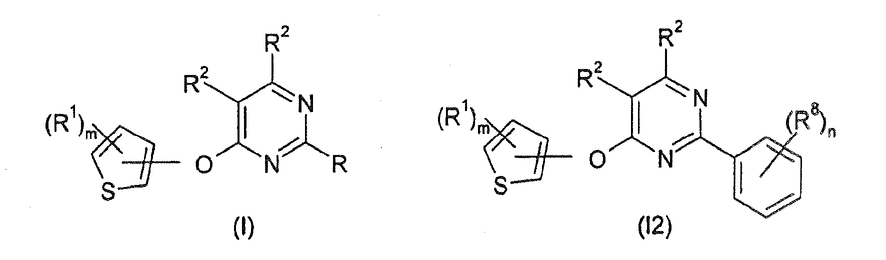 dans laquelle R, R¹, R² et m sont tels que définis dans les revendications 1 à 10, et R⁸ et n sont tels que définis dans la revendication 6, qui comporte la réaction d'un composé de formule II 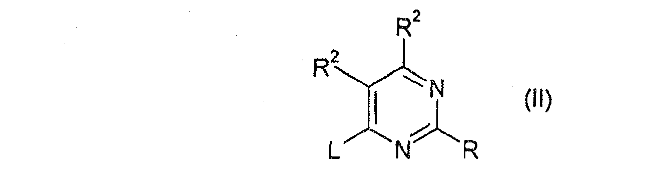 dans laquelle R et R² sont tels que définis ci-dessus pour la formule I ou 12, et L représente un groupe partant, avec un composé de formule III ou un sel métallique de celui-ci, 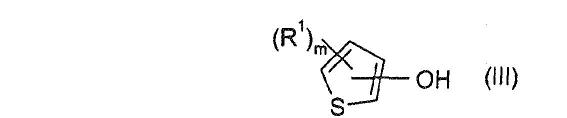 dans laquelle R¹ et m sont tels que définis ci-dessus pour la formule I.

11. Composé de formule IIIA 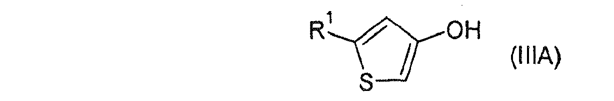 dans laquelle R¹ est tel que défini ci-dessus pour la formule I.

12. Composé selon la revendication 11, dans lequel R¹ représente un groupe trifluorométhyle.

13. Procédé pour la préparation d'un composé de formule IIIA 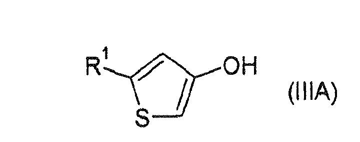 dans laquelle R¹ est tel que défini ci-dessus pour la formule I, qui comporte les étapes de
(a) saponification d'un composé de formule XIIIA 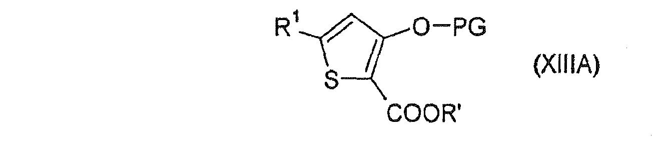 dans laquelle R¹ est tel que défini pour la formule IIIA ; R' est de l'alkyle en C₁₋₆ ; et PG représente un atome d'hydrogène ou un groupe de protection, pour obtenir un composé de formule XIVA 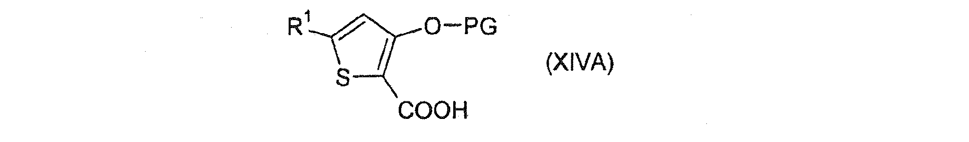
(b) décarboxylation du composé de formule XIVA ; et
(c) dissociation optionnelle du groupe de protection.

14. Composition herbicide constituée d'un support inerte solide ou liquide et d'une quantité herbicide efficace ayant la structure de la formule 1 ou 12 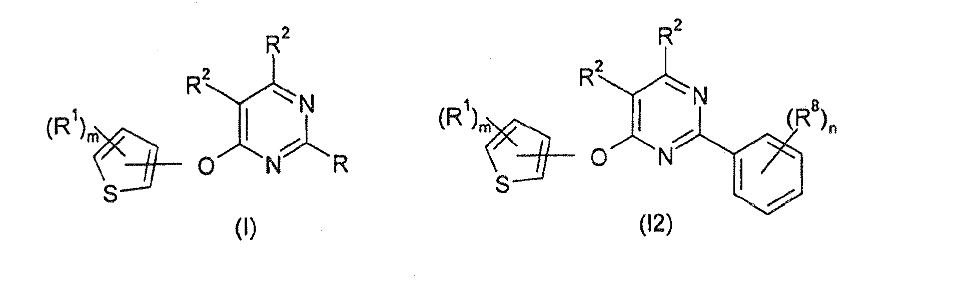 dans lesquelles R, R¹, R² et m sont tels que définis dans les revendications 1 ou 2, et R⁸ et n sont tels que définis dans la revendication 6.

15. Procédé pour le contrôle d'espèces végétales monocotylédones et dicotylédones annuelles, vivaces et aquatiques comportant l'application sur le feuillage desdites plantes, ou sur le sol ou dans l'eau contenant des graines ou d'autres organes de propagation de celles-ci, d'une quantité herbicide efficace du composé de formule I ou 12 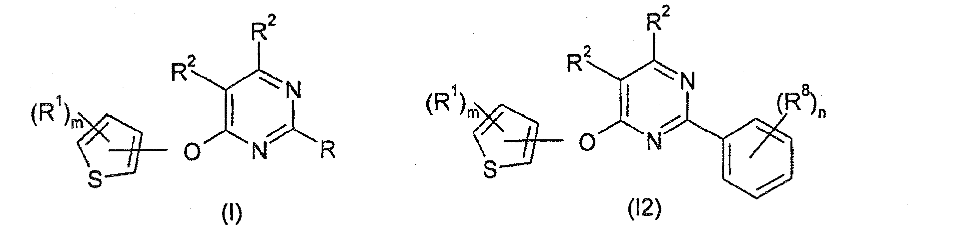 dans lesquelles R, R¹, R² et m sont tels que définis dans les revendications 1 à 4, et R⁸ et n sont tels que définis dans la revendication 6.
